(19) Europäisches Patentamt
European Patent Office
Office européen des brevets

(11) **EP 3 964 259 B1**

(12) **EUROPEAN PATENT SPECIFICATION**

(45) Date of publication and mention
of the grant of the patent:
**06.12.2023 Bulletin 2023/49**

(21) Application number: **21194598.5**

(22) Date of filing: **02.09.2021**

(51) International Patent Classification (IPC):
***A61N 1/375*** *(2006.01)*      ***A61N 1/37*** *(2006.01)*
***A61N 1/36*** *(2006.01)*

(52) Cooperative Patent Classification (CPC):
**A61N 1/375; A61N 1/36038; A61N 1/3718**

(54) **HOLDING MAGNETS AND MAGNET SYSTEM FOR IMPLANTABLE SYSTEMS OPTIMIZED FOR MRI**

HALTEMAGNETE UND MAGNETSYSTEM FÜR IMPLANTIERBARE SYSTEME, DIE FÜR MRT OPTIMIERT SIND

AIMANTS DE RETENUE ET SYSTÈME D'AIMANTS POUR SYSTÈMES IMPLANTABLES OPTIMISÉS POUR L'IRM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2020 EP 20194214**

(43) Date of publication of application:
**09.03.2022 Bulletin 2022/10**

(73) Proprietor: **MED-EL Elektromedizinische Geräte GmbH**
**6020 Innsbruck (AT)**

(72) Inventor: **ZIMMERLING, Martin**
**6082 Patsch (AT)**

(74) Representative: **Lucke, Andreas**
**Boehmert & Boehmert**
**Anwaltspartnerschaft mbB**
**Pettenkoferstrasse 22**
**80336 München (DE)**

(56) References cited:
**EP-A1- 2 560 730**        **EP-B1- 2 560 730**
**KR-A- 20200 078 292**   **US-B2- 9 872 993**
**US-B2- 10 532 209**

**Description**

FIELD OF THE INVENTION

The present invention relates to at least partially implantable devices such as partly implantable hearing devices, for example cochlear implants, and specifically, to implantable magnets for interaction with external magnets in such devices.

BACKGROUND ART

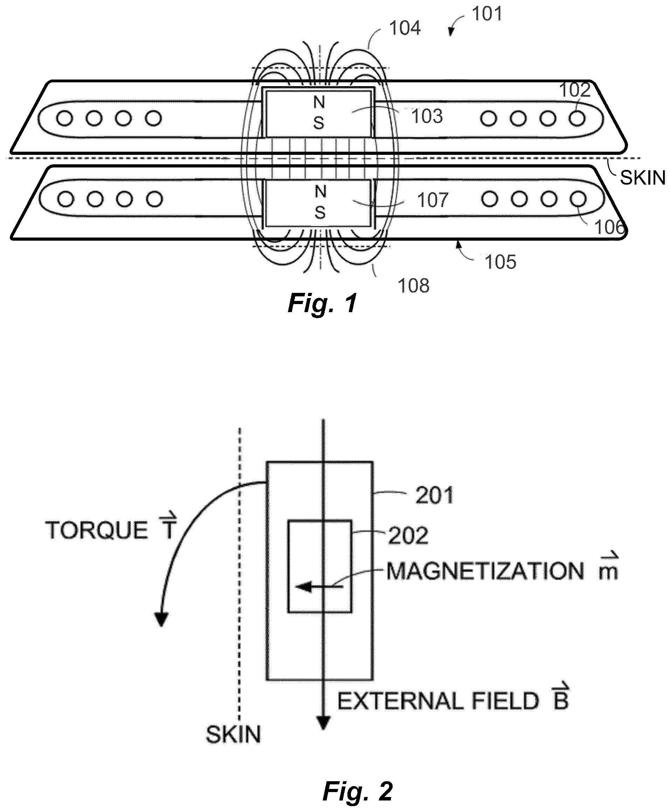

Some hearing implants such as Middle Ear Implants (MEI's) and Cochlear Implants (CI's) employ cooperating attachment magnets located in the implant and the external part to magnetically hold the external part in place over the implant. For example, as shown in Fig. 1, a typical cochlear implant system may include an external transmitter device **101** containing transmitting coil **102** and an external attachment magnet **103**. The external attachment magnet **103** has a conventional cylindrical disc-shape and a north-south magnetic dipole having an axis that is perpendicular to the skin of the patient to produce external magnetic field lines **104** as shown. Implanted under the patient's skin is a corresponding receiver assembly **105** having similar receiving coil **106** and an implant magnet **107**. The implant magnet **107** also has a cylindrical disc-shape and a north-south magnetic dipole having a magnetic axis that is perpendicular to the skin of the patient to produce internal magnetic field lines **108** as shown. The internal receiver device **105** is surgically implanted and fixed in place within the patient's body. The external transmitter device **101** is placed in proper position over the skin covering the internal receiver assembly **105** and held in place by interaction between the internal magnetic field lines **108** and the external magnetic field lines **104**. Rf signals from the transmitter coil **102** couple data and/or power to the receiving coil **106** which is in communication with an implanted processor module (not shown).

One problem arises when the patient undergoes Magnetic Resonance Imaging (MRI) examination. Interactions occur between the implant magnet and the applied external magnetic field for the MRI. As shown in Fig. 2, the direction of the magnetization $\vec{m}$ of the implant magnet **202** is essentially perpendicular to the skin of the patient. In this example, the strong static magnetic field $\vec{B}$ from the MRI creates a torque $\vec{T}$ on the internal magnet **202,** which may displace the internal magnet **202** or the whole implant device **201** out of proper position. Among other things, this may damage the adjacent tissue in the patient. In addition, the external magnetic field $\vec{B}$ from the MRI may reduce or remove the magnetization $\vec{m}$ of the implant magnet **202** so that it may no longer be strong enough to hold the external transmitter device in proper position. The implant magnet **202** also may cause imaging artifacts in the MRI image, there may be induced voltages in the receiving coil, and hearing artifacts due to the interaction of the external magnetic field $\vec{B}$ of the MRI with the implanted device. Torque and forces acting on the implant magnet and demagnetization of the implant magnet are especially an issue with MRI field strengths at 1.5 Tesla and higher. Thus, for many existing implant systems with magnet arrangements, it is common to either not permit MRI, or limit use of MRI to lower field strengths. Other existing solutions include use of a surgically removable magnets, spherical implant magnets (e.g. U.S. Patent 7,566,296), and various ring magnet designs (e.g., U.S. Patent Publication 2011/0022120).

U.S. Patent 8,634,909 discloses an implant magnet having a magnetic dipole moment direction that is parallel to the end surfaces of a disc shaped implant magnet-that is, perpendicular to the conventional magnetic dipole moment direction of a disc-shaped implant magnet. The magnet is then held in a magnet receptacle that allows the magnet to rotate about its center axis in response to an external magnetic field such as from an MRI to realign and minimize creating torque. But this rotation is only possible around a single axis.

It also has been suggested to use a set of multiple cylindrical magnets which are magnetized perpendicular to the cylinder axis and rotatable about their cylinder axis embedded into a magnet frame and case which can rotate around the central axis of the case (see e.g., WO2017/105510).

In that approach two or more diametrically-magnetized cylindrical magnets always align in a configuration where one north pole is oriented next to an adjacent south pole and vice versa. One disadvantage of this configuration is, that the cylindrical magnets together behave like a single disc shaped magnet as disclosed in the '909 patent with magnetization direction parallel to the skin surface - unless a very strong external magnetic field is applied -, but due to a relatively poor filling factor have only a small combined magnet volume, thus requiring an extra-large or extra-strong external magnet. On the other hand, using two or more cylindrical magnets does allow a relatively slim implant magnet design.

Further prior art is disclosed by US9872993B2, KR20200078292A, US10532209B2 and EP2560730A1.

## SUMMARY OF THE INVENTION

Embodiments of the present invention are directed to a magnet arrangement for a hearing implant device. An implant device contains signal processing circuitry configured for receiving an implant communications signal transmitted from an external transmitting coil through overlying skin of an implanted patient, and the implant device includes an outermost surface adapted to lie between the overlying skin and underlying skull bone of the implanted patient. There is a magnet case within the implant device, and the magnet case is configured to be rotatable about a case rotation axis which is at least approximately perpendicular to said outermost surface of the implant device. An implant magnet arrangement is located within the magnet case and configured to cooperate with a corresponding external holding magnet in an external device located over the overlying skin to magnetically hold the external device against the overlying skin. The implant magnet arrangement includes multiple cylindrical magnets, each with a center cylinder axis that is perpendicular to the case axis, and each cylindrical magnet is configured to be rotatable about its center cylinder axis. Each cylindrical magnet has an outer cylindrical surface with a north magnetic pole and a south magnetic pole, and a north magnetic direction is defined by a radial vector extending from the center cylinder axis to the north magnetic pole, and a south magnetic direction is defined by a radial vector extending from the south magnetic pole to the center cylinder axis. The north magnetic pole and the south magnetic pole are arranged with respect to each other so as not to lie on a common diameter through the center cylinder axis such that the north magnetic direction and the south magnetic direction form a magnetic angle less than 180 degrees with a vertex at the center cylinder axis.

In further specific embodiments, the implant magnet arrangement may be configured for the cylindrical magnets to magnetically align with respect to each other to create a common line of magnetic flux through the cylindrical magnets, the magnet case, and the overlying skin to cooperate with the external holding magnet. There also may be one or more diametrically magnetized supplemental cylindrical magnets located between the cylindrical magnets and configured to couple the common line of magnetic flux between the cylindrical magnets. In addition or alternatively, there may be soft magnetic material located between the cylindrical magnets and configured to couple the common line of magnetic flux between the plurality of cylindrical magnets.

The implant magnet arrangement may be configured to respond to a strong external magnetic field by rotation of the magnet case about the case rotation axis and rotation of the cylindrical magnets about their respective center cylinder axes so as to minimize net torque imparted to the implant device. The magnetic angle may be between 90 degrees and 140 degrees. Each cylindrical magnet may be configured to be fully rotatable about the center cylinder axis through a complete rotation range of 360 degrees. Or each cylindrical magnet may be configured to be limitedly rotatable about the center cylinder axis through a limited rotation range of less than 180 degrees. For example, the limited rotation range may be 90 degrees.

A second aspect of the invention relates to an implant device containing signal processing circuitry configured for receiving an implant communications signal transmitted (e.g. from an external transmitting coil) through overlying skin of an implanted patient, wherein the implant device includes an outermost surface adapted to lie between the overlying skin and underlying skull bone and at least approximately parallel to the skin of the implanted patient; and an implant magnet configured to cooperate with an external holding magnet in an external device to be located over the overlying skin to magnetically hold the external device against the overlying skin. According to the second aspect of the invention, said implant magnet has a north magnetic pole, a south magnetic pole, and as a whole has an overall magnetic dipole moment that is parallel to or at an angle of 30° or less, preferably 20° or less with respect to said outermost surface. Moreover, said implant magnet has a north end portion including said north magnetic pole and a south end portion including said south magnetic pole, said north and south end portions each being formed from permanent magnetic material and each having an individual magnetic dipole moment that is inclined with respect to said overall magnetic dipole moment, wherein said individual magnetic dipole moment in said north end portion is inclined with respect to said overall magnetic dipole moment such as to have a component pointing towards said outermost surface, and said individual magnetic dipole moment in said south end portion is inclined with respect to said overall magnetic dipole moment such as to have a component pointing away from said outermost surface. The north and south end portions of the implant magnet may be fixedly attached directly with each other, or may each be fixedly attached to an intermediate portion of said implant magnet.

According to the second aspect of the invention, the implant magnet "as a whole" has an "overall magnetic dipole moment" which is at least approximately parallel to the outermost surface, or in other words, approximately parallel to the skin in the implanted state. As the skilled person will appreciate, the magnetic dipole moment $m$ of a magnetic body as a whole is a macroscopic quantity defining the "strength" of the magnetic dipole. When arranged in an external magnetic field having a flux density $B$, a torque $\tau$ is generated that corresponds to the vector product of the magnetic dipole moment $m$ and the flux density $B$, i.e. $\tau = m \times B$. Accordingly, the overall magnetic dipole moment $m$ of the implant magnet can be readily determined when placed in an external magnetic field: the implant magnet will orient itself to bring the dipole moment $m$ in alignment with the flux density $B$ of the external magnetic field, thereby revealing the direction of the magnetic dipole moment $m$, while its magnitude is defined by the size of the torque needed to rotate the implant magnet out of this alignment. The magnetic dipole moment of the implant magnet as a whole determines how the internal magnet reacts to the external magnetic field in an MRI device.

However, according to the invention, the implant magnet has north and a south end portions including the north and south magnetic poles, respectively, each having an individual magnetic dipole moment that is inclined with respect to said overall magnetic dipole moment. In these north and south end portions, the respective magnetizations are hence not aligned with the overall magnetic dipole moment of the implant magnet as a whole. The magnetization of a material is designated as a vector field $\boldsymbol{M}$ that expresses the density of magnetic dipole moments in the magnetic material, i.e.

$$\boldsymbol{M} = \frac{d\boldsymbol{m}}{dV},$$

, where $d\boldsymbol{m}$ is the elementary magnetic moment and $dV$ is the corresponding volume element. In other words, the magnetic moment $\boldsymbol{m}$ associated with a magnet is the space integral of the magnetization $\boldsymbol{M}$ over the magnet's volume, i.e. $\boldsymbol{m} = \iiint \boldsymbol{M}\, dV$. As used herein, a "vector" is simply understood as a physical object that has a magnitude and a direction. No distinction between vectors and pseudovectors according to their transformation properties is made herein. Vectors are generally represented by symbols in bold font.

In the invention, the north and south end portions are each formed from permanent magnetic material. A "permanent magnetic material" as understood herein has a broad meaning, but is in any case distinguished from magnetic soft materials such as soft iron as it is used in the art for pole shoes or the like. In particular, the permanent magnetic material in the north and south end portions should have an intrinsic magnetic coercivity $H_{Ci}$ of more than 200 A/m, preferably of more than 500 A/m, more preferably more than 800 A/m and most preferably more than 1000 A/m. The individual magnetic dipole moment in said north end portion has a component pointing towards said outermost surface, and the individual magnetic dipole moment in said south end portion has a component pointing away from said outermost surface. The inventor has found that this way, the magnetic attraction or holding force applied to an external magnet of an external device can be significantly increased as compared to a prior art implant magnet of same size and material which is homogeneously magnetized in a direction parallel to the skin throughout its volume, while still allowing for an overall magnetic moment that is parallel to the skin.

In preferred embodiments, the implant magnet is rotatable around a rotation axis that is perpendicular to said outermost surface, or deviates from perpendicular by less than 30°, preferably less than 20°, wherein in each available rotational position of said implant magnet upon rotation around its rotation axis, said overall magnetic dipole moment is parallel to or at an angle of 30° or less, preferably 20° or less with respect to said outermost surface.

Preferably, said implant magnet has a shape that is rotationally symmetric around said rotation axis. In a preferred embodiment, said implant magnet has an outer end surface facing said outermost surface of said implant device and an inner end surface facing away from said outermost surface, wherein one or both of said inner and outer end surfaces are perpendicular to said rotation axis. For example, the implant magnet may have a disc shape.

In preferred embodiments, said implant magnet has a planar outer end surface, to optimally make use of the limited space in the implant device.

In a preferred embodiment, the angle of inclination between each individual magnetic dipole moment in said north and south end portions with respect to the overall magnetic dipole moment is $\leq 50°$. This will allow for safely avoiding a situation in which the implant magnet could be inadvertently weakened or de-magnetized in a strong external MRI field in case the patient does not hold his or her head straight during the MRI procedure, but for example tilted to one side by e.g. up to 30°.

In a preferred embodiment, said implant magnet has an average diameter $d_I$ in a direction parallel to the overall magnetic dipole moment and an average thickness $h_I$ in a direction perpendicular to said outermost surface, wherein in one or both of said north and south end portions, said individual magnetic dipole moment is inclined with respect to said overall magnetic dipole moment by an angle a, wherein

$$arctan\,(h_I\,/\,(d_I\,/2))\,\text{-}15° \leq \alpha \leq arctan\,(h_I\,/\,(d_I\,/2))\,+\,7°,$$

preferably

$$arctan\,(h_I\,/\,(d_I\,/2))\,\text{-}10° \leq \alpha \leq arctan\,(h_I\,/\,(d_I\,/2))\,+\,5°.$$

Herein, the angle $\alpha$ is measured in a plane that is perpendicular to the outermost surface. This angular range has been found to allow for a particularly good increase in the attachment force. For larger angles a, the distance between the north and south poles would decrease, which in turn would lead to an excessive decrease in holding force with distance from the implant magnet.

In some embodiments, said north and south end portions are directly adjacent with each other, and in particular each form one of two halves of said implant magnet. This embodiment leads to very good holding forces, and at the same time allows for comparatively easy manufacture.

In alternative embodiments, however, said north and south end portions of said implant magnet may be separated from each other by an intermediate portion having an individual magnetic dipole moment that is parallel to said overall magnetic dipole moment, or deviates from parallel by less than 10°, preferably less than 5°. This embodiment allows for comparatively large inclinations of the magnetization in the north and south end portions while at the same time avoiding magnetic short-circuits at the outer surface, thereby leading to very good holding forces.

In addition or alternatively, one or both of said north and south end portions of said implant magnet may have an outer section closer to said outermost surface and an inner section further away from said outermost surface, wherein an angle of inclination of the individual magnetic dipole moment with respect to the overall magnetic dipole moment in said outer section is less than in said inner section. This embodiment allows for an improved magnetic flux within the implant magnet while avoiding the distance of the north and south poles to decrease.

In a preferred embodiment, said implant magnet has an outer end surface facing said outermost surface of said implant device and an inner end surface facing away from said outermost surface. Moreover, a middle plane is defined to be located at equal distance from said outer and inner end surfaces, and said implant magnet preferably fulfils one or both of the following criteria (i) and (ii):

(i) at least 55 %, preferably at least 65 % of the total magnetic flux of a magnetic field generated outside of the implant magnet when placed in isolation in air or a vacuum is located on a side of said middle plane at which said outermost surface is located in the assembled state,

(ii) more than 50%, preferably more than 55% of the mass of the magnet is located on a side of said middle plane at which said outermost surface is located, wherein in particular, the edges of the magnet at the inner end surface are chamfered.

Criterion (i) defines the distribution of the magnetic flux generated by the implant magnet itself, i.e. when placed in isolation in air or vacuum. According to this criterion, the larger part of the magnetic flux is on one side of the middle plane, and this side is the side on which in the "assembled state", i.e. when the implant magnet is arranged in the implant device, the outermost surface of the implant device would be located. In other words, the implant magnet is devised so that by itself it already generates the bigger part of its flux in the region where it is needed for establishing an attractive holding force with an external device, i.e. more to the outside than to the inside of the body.

The second criterion (ii) specifies that more than half of the mass of the implant magnet is located on the side of the middle plane at which the outermost surface located. This again helps with generating the magnetic flux closer to the outside region than the inside region with respect to the implant magnet. One way of reducing the mass of the implant magnet towards the inside of the middle plane is by providing for chamfered edges at the inner end surface of the implant magnet. This shape also allows for a more favorable magnetic flux.

In a preferred embodiment, said north and south end portions are formed from anisotropic magnet elements each having a preferred magnetization direction, wherein said anisotropic magnet elements are joined with each other or with an intermediate portion arranged in between. Said preferred magnetization directions are arranged at an angle with respect to the overall dipole moment of the implant magnet as a whole. The anisotropic magnet elements can e.g. be made by applying an external magnetic field while the magnet is formed, which may for example involve sintering. The anisotropic magnets have the advantage that they allow for higher magnetizations in their final state. The anisotropic magnets will not have their final magnetic strength after manufacture yet, but will only acquire it after a final magnetization using a strong magnetic pulse. While an isotropic magnet can be magnetized by a strong magnetic pulse in any direction, the anisotropic magnets can only be magnetized in their preferred magnetization directions established upon manufacture. This is actually an advantage in the manufacturing process of the magnet as a whole, because the magnet can be assembled from anisotropic magnet elements with the preferred magnetization directions in the north and south end portions inclined with respect to the overall magnetization direction, but before the anisotropic magnet elements are fully magnetized. This makes the handling of the anisotropic magnet elements prior to the assembly, as well as the joining of the magnet elements much easier. It is then possible to fully magnetize the anisotropic magnet elements in the joint state by applying an external magnetic pulse aligned with the direction of the eventual overall magnetic dipole moment. During this magnetization process, the magnetization directions of the anisotropic magnet elements as part of the entire implant magnet will be preserved, while their strength will be increased.

In preferred embodiments, said implant magnet may have on at least part of the inner end surface (913) a layer of magnetic soft material, such as for example soft iron, applied. This may help to shield a magnetic field generated by the implant magnet itself toward the inside of the body and thereby may reduce artefacts during MRI-scanning.

In preferred embodiments, said implant magnet is a rare earth magnet, in particular a rare earth magnet comprising neodymium, samarium, terbium, dysprosium or holmium.

A further embodiment of the invention relates to an implant system comprising an implant device or magnet arrangement according to one of the preceding embodiments and an external device containing signal processing circuitry configured for transmitting an implant communications signal to said implant device, said external device comprising an innermost surface adapted to lie adjacent to said skin; and an external magnet or magnet assembly in said external device to be located over the overlying skin and magnetically configured to cooperate with an implant magnet or magnet arrangement as defined in any of the preceding embodiments such as to hold the external device against the skin.

The external magnet may be similar to the implant magnet, but may also be of a different design. In particular, since the external device can be taken off prior to an MRI procedure, the external magnet does not have to be specifically designed to cope with strong external MRI magnetic fields. Accordingly, the external magnet may be an arrangement of two magnets having magnetic dipole moments arranged perpendicular to the innermost surface of the external device, and hence to the skin, with their north and south poles arranged adjacent to the south and north poles of the implant magnet, respectively.

However, in some embodiments of the implant system, said external magnet or magnet arrangement may have a north magnetic pole, a south magnetic pole, and may further as a whole have an overall magnetic dipole moment that is parallel to or at an angle of 30° or less with respect to said innermost surface of said external device.

Moreover, the external magnet may be of a similar design as disclosed in one of the embodiments with respect to the internal magnet above. In particular, the external magnet may have a north end portion including said north magnetic pole and a south end portion including said south magnetic pole, said north and south end portions each being formed from permanent magnetic material and each having an individual magnetic dipole moment that is inclined with respect to said overall magnetic dipole moment of said external magnet, wherein said individual magnetic dipole moment in said north end portion has a component pointing towards said innermost surface of said external device, and said individual magnetic dipole moment in said south end portion has a component pointing away from said innermost surface of said external device.

In a preferred embodiment of said implant system, said external magnet is rotatable around a rotation axis that is perpendicular to said innermost surface of said external device or deviates from perpendicular by less than 30°, wherein in each available rotational position of said external magnet upon rotation around its rotation axis, said overall magnetic dipole moment is parallel to or at an angle of 30° or less with respect to said innermost surface, wherein said external magnet preferably has a shape that is rotationally symmetric around its rotation axis.

In preferred embodiments, said external magnet has a planar inner end surface facing said innermost surface of said external device.

In a preferred embodiment of the implant system, said external magnet has an average diameter $d_E$ in a direction parallel to the overall magnetic dipole moment and an average thickness $h_E$ in a direction perpendicular to said innermost surface of the external device, wherein in one or both of said north and south end portions, said individual magnetic dipole moment is inclined with respect to said overall magnetic dipole moment by an angle a, wherein

$$arctan\,(h_E\,/\,(d_E\,/2))\,\text{-}15° \leq \alpha \leq arctan\,(h_E\,/\,(d_E\,/2))\,+\,7°,$$

preferably

$$arctan\,(h_E\,/\,(d_I\,/2))\,\text{-}10° \leq \alpha \leq arctan\,(h_E\,/\,(d_E\,/2))\,+\,5°.$$

The advantages of these angular ranges are similar to those explained above with reference to the implant magnet. Similar to the case of the implant magnet, the angle $\alpha$ is measured in a plane that is perpendicular to the innermost surface of the external device, or in other words, in a plane that is at least approximately perpendicular to the skin.

In some embodiments, said north and south end portions of said external magnet are directly adjacent with each other, and in particular each form one of two halves of said external magnet. In alternative embodiments, said north and south end portions of said external magnet are separated from each other by an intermediate portion having an individual magnetic dipole moment that is parallel to said overall magnetic dipole moment of said external magnet or deviates from parallel by less than 10°, preferably less than 5°.

In a preferred embodiment of the implant system, one or both of said north and south end portions of said implant magnet have an inner section closer to said innermost surface of said external device and an outer section further away from said innermost surface of said external device, wherein an angle of inclination of the individual magnetic dipole moment with respect to the overall magnetic dipole moment in said inner section is less than in said outer section.

In a preferred embodiment, said external magnet has an inner end surface facing said innermost surface of said external device and an outer end surface facing away from said innermost surface of said external device, wherein a middle plane is defined to be located at equal distance from said outer and inner end surfaces of said external device, and wherein said external magnet fulfils one or both of the following criteria (i) and (ii):

(i) at least 55 %, preferably at least 65 % of the total magnetic flux of a magnetic field generated outside of the external magnet when placed in isolation in air or a vacuum is located on a side of said middle plane at which said innermost surface of the external device is located in the assembled state,
(ii) more than 50%, preferably more than 55% of the mass of the external magnet is located on a side of said middle plane at which said innermost surface is located, wherein in particular, the edges of the magnet at the inner end surface are chamfered.

In a preferred embodiment, said north and south end portions of said external magnet are formed from anisotropic magnet elements each having a preferred magnetization direction, wherein said anisotropic magnet elements are joined with each other or with an intermediate portion arranged in between, wherein said preferred magnetization directions are arranged at an angle with respect to the overall dipole moment of the external magnet as a whole.

In a preferred embodiment, said external magnet is a rare earth magnet, in particular a rare earth magnet comprising neodymium, such as neodymium-ion-boron magnets, or comprising samarium, such as samarium-cobalt magnets, or comprising terbium, or dysprosium or holmium or combinations thereof.

Embodiments of the present invention also include a hearing implant system containing a magnet arrangement according to any of the foregoing.

BRIEF DESCRIPTION OF THE DRAWINGS

Figure 1 shows portions of a typical cochlear implant system and the magnetic interaction between the implant magnet and the external implant magnet.

Figure 2 illustrates the force interactions that can occur between an implant magnet and the applied external magnetic field for an MRI system.

Figure 3A shows an example of a cylindrical implant magnet with a V-shaped magnetic angle according to an embodiment of the present invention.

Figure 3B shows an example of a cochlear implant device with two implant magnets of the type shown in Fig. 3A.

Figures 4A and 4B show how the magnetic fields of the implant magnets align to cooperate with an external holding magnet.

Figures 5A and 5B show how the magnetic fields of the implant magnets align in the presence of an MRI magnetic field.

Figures 6A and 6B show an example of an embodiment with a diametrically magnetized supplemental cylindrical magnet located between the cylindrical magnets.

Figures 7A and 7B show an example of an embodiment with soft magnetic material located between the cylindrical magnets.

Figure 8 shows an embodiment of cylindrical implant magnets that are configured to be limitedly rotatable through a limited rotation range according to an embodiment of the present invention.

Figures 9A-9C show orientations of the cylindrical implant magnets for different orientations of an external MRI magnetic field.

Figures 10A-10C show orientations of the cylindrical implant magnets for reverse magnetization in a reduced rotatability embodiment of the present invention.

Figure 11 shows a further example of a cochlear implant device using an implant magnet with north and south end portions having individual magnetic dipole moments that are inclined with respect to the overall magnetic at the moment of the implant magnet.

Figure 12 is a perspective view of an implant magnet of the type used in the device of Figure 11.

Figure 13 is a side view of the device of Figure 11.

Figure 14 is a schematic sectional view showing an implant device and an external device with corresponding magnets.

Figure 15 is a schematic sectional view showing magnets of an implant and an external device.

Figure 16 illustrates the direction of the magnetic flux generated by an ordinary implant magnet that is homogeneously magnetized.

Figure 17 illustrates the direction of the magnetic flux generated by an implant magnet of the invention having north and south end portions having individual magnetic dipole moments that are inclined with respect to the overall magnetic dipole moment of the implant magnet.

Figure 18 illustrates the flux density of the ordinary implant magnet of Figure 16.

Figure 19 illustrates the flux density of the implant magnet of the invention according to Figure 17.

Figure 20 shows the magnetic flux density generated upon the interaction of a homogeneously magnetized external magnet and the ordinary implant magnet of Figure 16.

Figure 21 shows the magnetic flux density generated upon the interaction of homogeneously magnetized external magnet and the implant magnet of the invention according to Figure 17.

Figure 22 is a schematic sectional view of an implant magnet and an external magnet according to a further embodiment of the invention.

Figure 23 is a schematic view illustrating the torque applied to a conventional implant magnet and an implant magnet of the invention by an external magnetic field.

DETAILED DESCRIPTION OF SPECIFIC EMBODIMENTS

A larger distance between the two magnetic poles has the advantage that the attractive magnetic force for an external magnet does not decrease so steeply with increasing distance between the magnets. Embodiments of the present invention are directed to an improved implant magnet arrangement that uses two cylindrical implant magnets with magnetization direction having a V-shaped magnetic angle. These magnets are mounted in the implant device such that a "strong" side (i.e. the side with high magnetic flux) faces at least partly towards the overlying skin. Both magnets are mounted inside a magnet case in which they can turn also around the rotation axis of the case.

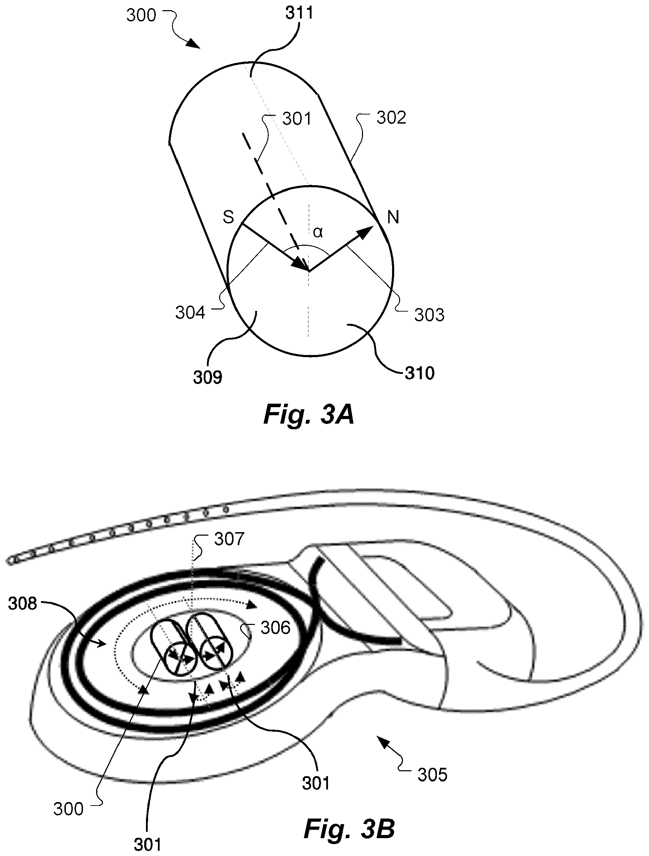

Figure 3A shows an example of a cylindrical implant magnet **300** with a V-shaped magnetic angle $\alpha$ according to an embodiment of the present invention, and Figure 3B shows an example of a cochlear implant device **305** with two implant magnets **300** of the type shown in Fig. 3A. The cochlear implant device **305** contains signal processing circuitry (not shown) configured for receiving an implant communications signal transmitted from an external transmitting coil through overlying skin of an implanted patient, and an outermost surface **308** adapted to lie between the overlying skin and underlying skull bone of the implanted patient.

There is a magnet case **306** within the implant device **305** with a case rotation axis **307** that is perpendicular to the outermost surface **308** of the implant device **305.** The magnet case **306** is configured to be rotatable about the case rotation axis **307.** Typically the magnet case **306** is surrounded by a receiver coil of the implant device **305.** The magnet case **306** may be metallic (e.g. made of titanium), or it may be made of a biocompatible non-metallic material (e.g. PEEK, FEP, PTFE, PSU, etc.) and may be coated (e.g. with Parylene). The magnet case **306** may be adapted to facilitate long-term hermetic encapsulation, and/or it may be adapted to be surgically removable for minimized susceptibility to MRI artifacts.

An implant magnet arrangement includes multiple cylindrical magnets **300** located within the magnet case **306** and configured to cooperate with a corresponding external holding magnet in an external device located over the overlying skin to magnetically hold the external device against the overlying skin. Each cylindrical implant magnet **300** has a center cylinder axis **301** that is perpendicular to the case rotation axis **307,** and each cylindrical magnet **300** is configured to be rotatable about its center cylinder axis **301.**

Each cylindrical magnet **300** has an outer cylindrical surface **302** with a north magnetic pole and a south magnetic pole. In the most general sense, a "cylindrical surface" is a surface consisting of all the points on all the lines which are parallel to a reference line and which pass through a fixed plane curve in a plane not parallel to the given line. In the present disclosure, the cylinder is a so-called right circular cylinder, in which the "fixed plane curve" is a circle, and the reference line is a line that is perpendicular to circle plane, for example the center cylinder axis 301. A north magnetic direction **303** is defined by a radial vector extending from the center cylinder axis **301** to the north magnetic pole. And a similar south magnetic direction **304** is defined by a radial vector extending from the south magnetic pole to the center cylinder axis **301**. The north magnetic pole and the south magnetic pole are arranged with respect to each other so as not to lie on a common diameter through the center cylinder axis **301** such that the north magnetic direction **303** and the south magnetic direction **304** form a "magnetic angle" $\alpha$ that is less than 180 degrees with a vertex at the center cylinder axis **301**. For example, the magnetic angle $\alpha$ may specifically be between 90° and 140° (or some other defined range). Such magnetic angle $\alpha$ can be established for example by forming the cylindrical magnet **300** from two preformed portions **309** and **310** which are magnetized according to the above-mentioned north and south magnetic directions **303, 304** in a manner that will be explained with reference to a further embodiment in more detail below. In the embodiment shown in Fig. 3, the preformed portions **309, 310** each correspond to a longitudinal half of the full cylindrical magnet **300** which are attached to each other along an interface indicated by the dashed line **311** in Fig. 3A.

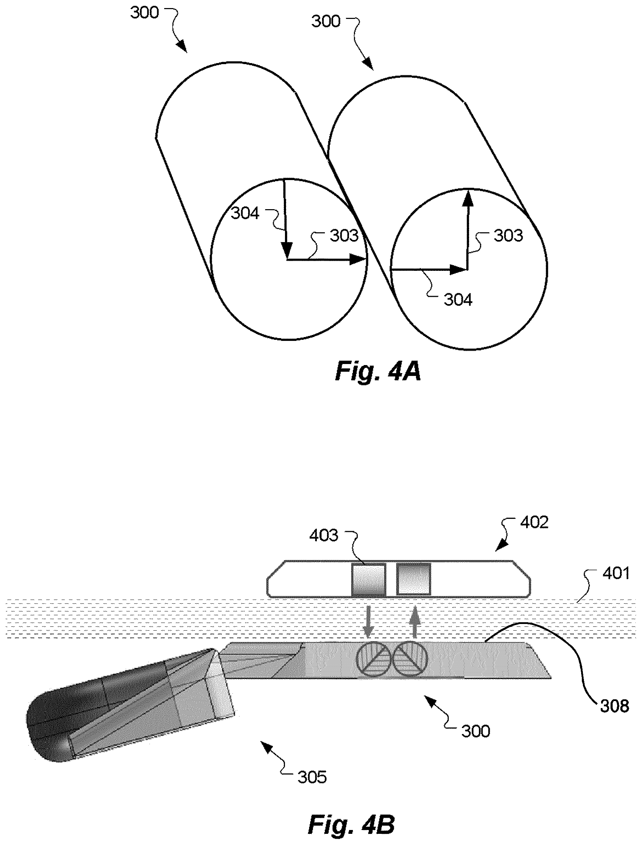

Figures 4A and 4B show how the magnetic fields of the cylindrical magnets 300 align to cooperate with one or more external holding magnets **403** in an external device **402** where the cylindrical magnets **300** magnetically align with respect to each other to create a common line of magnetic flux through the magnets, the magnet case, and the overlying skin to cooperate with the external holding magnet 403. Since the two cylindrical magnets **300** are arranged closely together (e.g., less than 2 mm between their outer cylindrical surfaces **302**), the two adjacent magnetic poles form an attractive magnetic connection with a common magnetic direction that is parallel to the outer surface **308** of the implant device **305** and the overlying skin **401**. Due to the V-shaped magnetic angle, the magnetic direction of the non-adjacent halves of the two cylindrical magnets **300** guides the magnetic flux towards the outer surface **308** of the implant device **305** and the overlying skin **401,** thereby allowing a strong magnetic attraction with the external holding magnet **403** that is almost as strong as with older-type axially magnetized implant magnets that have a magnetization direction that is also normal to the skin **401**.

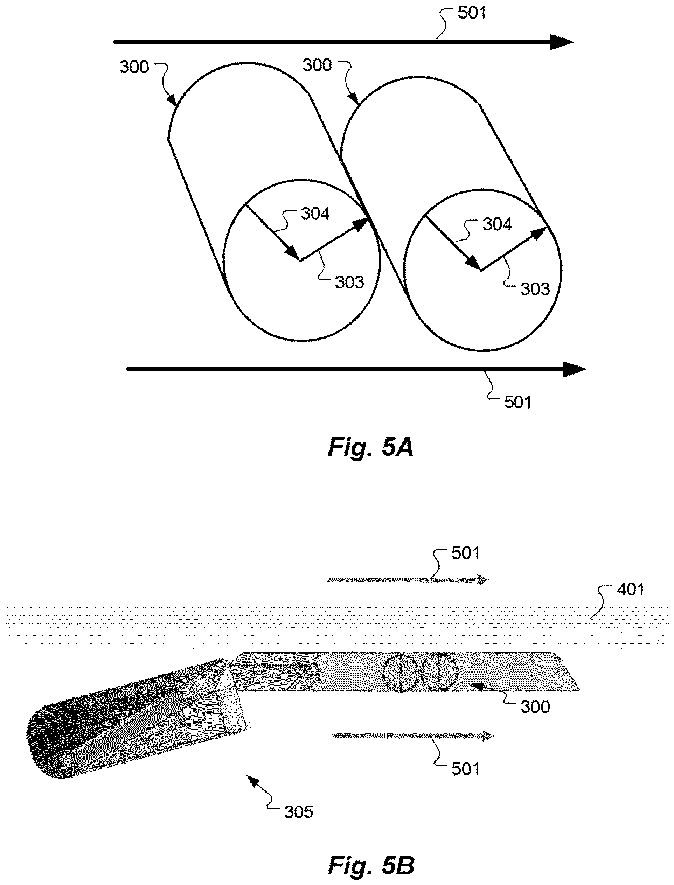

Figures 5A and 5B show how the cylindrical magnets **300** align in the presence of an MRI magnetic field **501**. As can be seen, the two cylindrical magnets **300** immediately align relative to the external magnetic field **501** so that the torque generated by the respective magnetizations of the different halves of each magnet **300** interacting with the external magnetic field **501** cancels out. In Fig. 5B to 7B, the magnetization is schematically represented by the hatched lines.

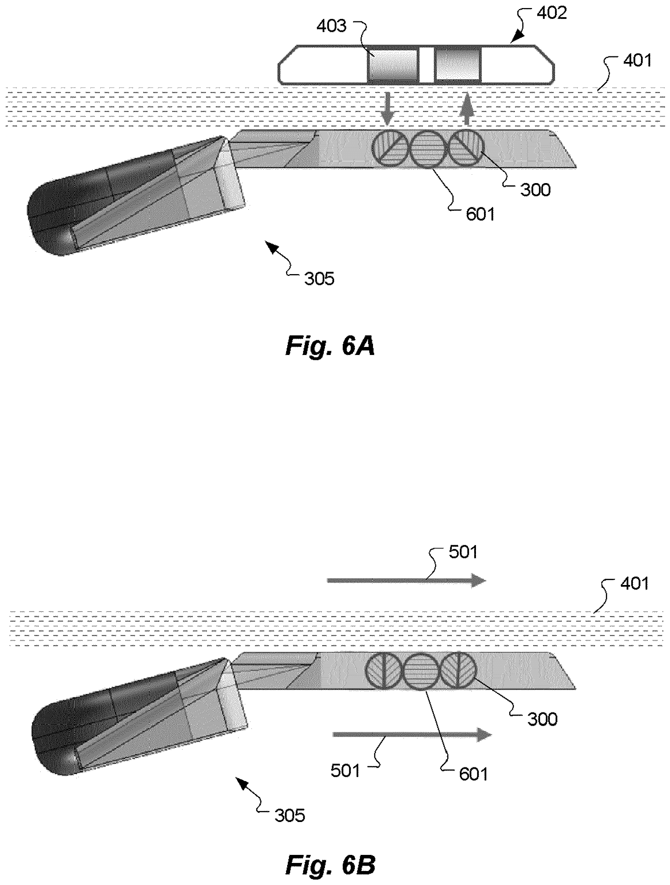

Figures 6A and 6B show an example of an embodiment with a diametrically magnetized supplemental cylindrical magnet **601** that is located between the cylindrical magnets 300. The supplemental cylindrical magnet **601** is configured to couple the common line of magnetic flux between the cylindrical magnets **300**. This provides an increased distance between the two local maxima of the magnetic flux through the skin **401** thus improving the magnetic attraction to the external magnet **403**.

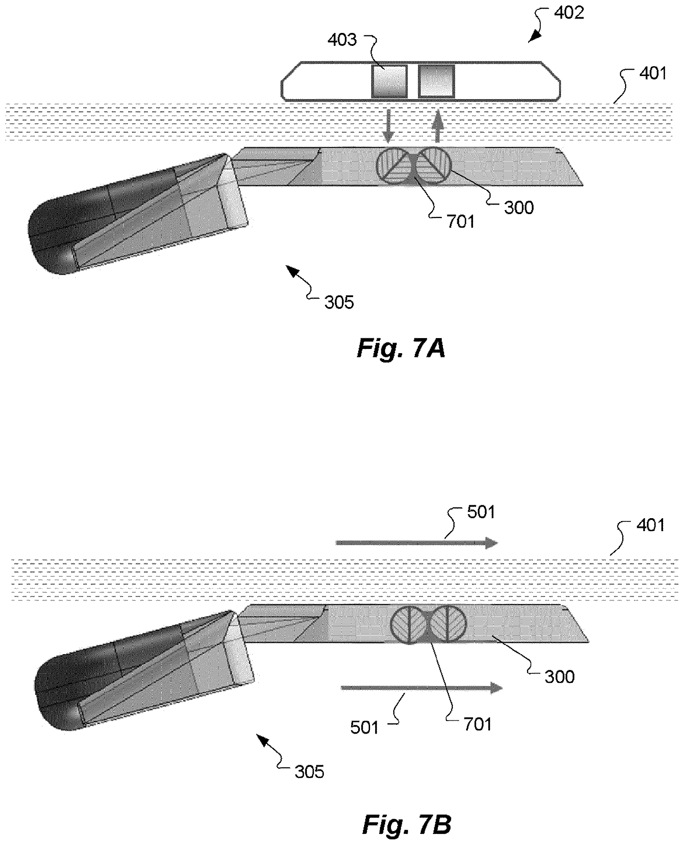

Figures 7A and 7B show an example of an embodiment with soft magnetic material **701** that is located between the cylindrical magnets **300** to couple the common line of magnetic flux between the cylindrical magnets **300**.

In the embodiments described above, the cylindrical magnets **300** are configured to be fully rotatable about the center cylinder axis **301** through a complete rotation range of 360 degrees, and so that the magnet case **306** containing the cylindrical magnets **300** can turn around its case axis **307**. Otherwise, when the center cylinder axis **301** were fixed and a strong external magnetic field **501** is oriented anti-parallel to the cylindrical magnets **300,** the magnets would flip by 180° and the magnetically-strong side of the magnets would then face towards the underlying skull in a medial direction instead of towards the skin in lateral direction. Note that the orientation of the external magnetic field **501** in a given MRI scanner is different when an implant user is scanned with the head first versus with legs first. Accordingly, it is advantageous if the magnet arrangement can handle both of these orientations of the external magnetic field **501**. Moreover, there is no general convention of the orientation of the external magnetic field **501** in MRI scanners, and in some cases where two MRI scanners are arranged next to each other in the same facility, the orientations of the respective external magnetic fields **501** are even deliberately chosen to be of opposite orientation.

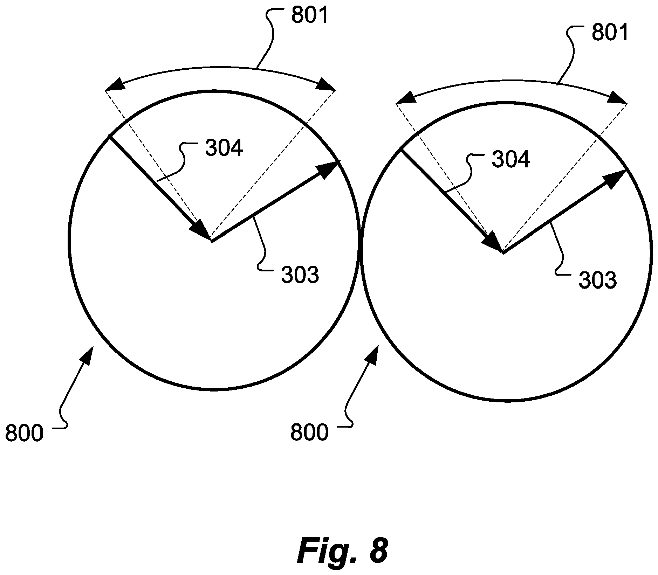

Still, a further design variant with two cylindrical magnets in V-shaped magnetization also works when the magnets have only one degree of freedom and have a rotation angle limited to about 90° only. Figure 8 shows an embodiment of cylindrical implant magnets **800** that are configured to be limitedly rotatable through a limited rotation range **801** of less than 180 degrees according to another embodiment of the present invention. For example, the cylindrical implant magnets may have a V-shaped magnetization with a magnetic angle between 100° and 140°, and the limited rotation range **801** may be 90°.

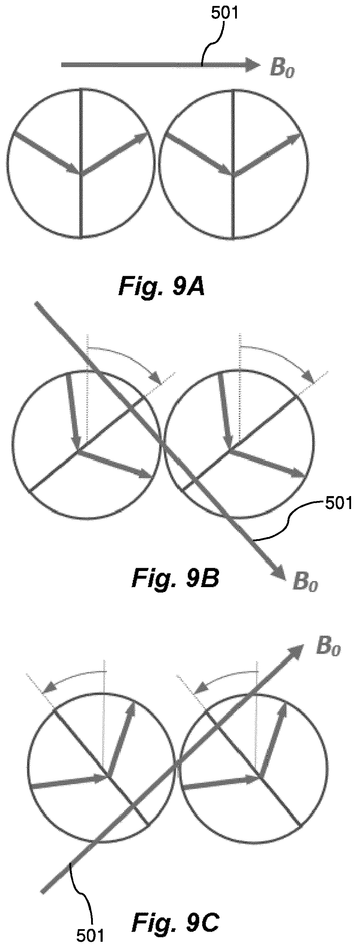

Figures 9A-9C show orientations of the limited rotation range cylindrical implant magnets **800** for different orientations of an external MRI magnetic field **501**. As long as the strong external magnetic field **501** has a component oriented parallel to the overall magnetization of the cylindrical implant magnets **800**, the magnets behave the same as with the other embodiments described above.

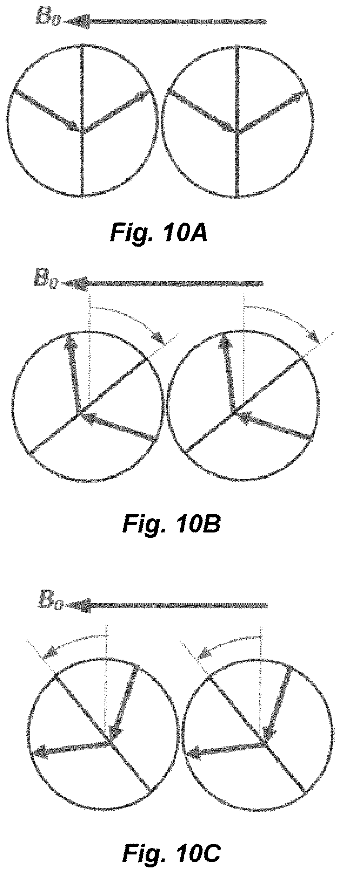

Figures 10A-10C show orientation of the limited range cylindrical implant magnets for reverse magnetization in a reduced rotatability embodiment of the present invention. When a strong external magnetic field is oriented antiparallel to the overall magnetization of the cylindrical implant magnets **800,** they cannot turn by roughly 180° to align to be parallel with the external magnetic field. Instead, each implant magnet **800** reverses its magnet polarity.

In the embodiments described above, despite some individual parts of the implant magnet being oriented perpendicular to the skin surface, the magnets do not weaken in an MRI environment because they immediately turn into a safe orientation relative to the strong static magnetic field of the MRI scanner. Each individual magnet always has a component parallel to the strong static magnetic field of the MRI scanner. And so each individual magnet always aligns such that there is no torque to the outside. The magnetic flux is directed to the skin side and reduced in medial direction. Therefore, the MRI artefact reaches less into the medial direction and is more oriented towards the skin side.

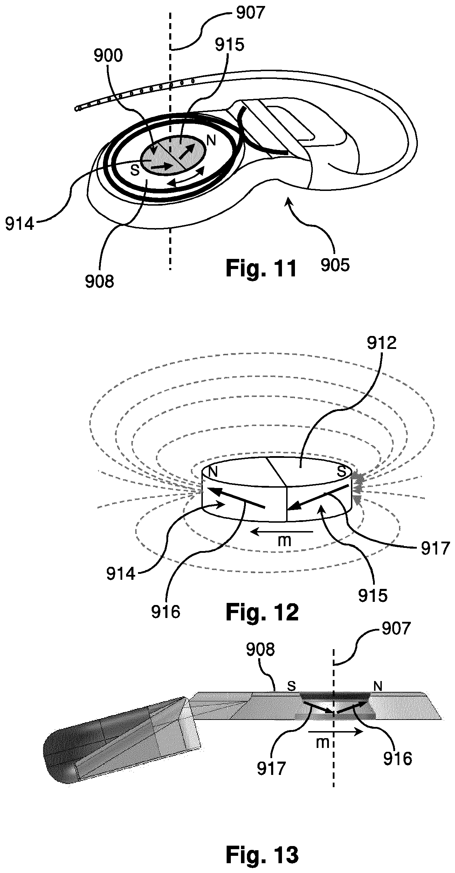

Shown in Fig. 11 is an example of a further implant device, in the specific embodiment a cochlear implant device **905** that is generally similar to the cochlear implant device **305** of Fig. 3B. The cochlear implant device **905** contains signal processing circuitry (not shown) configured for receiving an implant communications signal transmitted from an external device, such as an external device as shown under reference sign **402** in figures 4B and 6A through overlying skin 401 of an implanted patient.

The implant device **905** includes an outermost surface **908** adapted to lie between the overlying skin **401** and underlying skull bone and is at least approximately parallel to the skin **401** of the implanted patient. The implant device **905** further comprises an implant magnet **900** configured to cooperate with an external holding magnet in an external device **402** to be located over the overlying skin **401** to magnetically hold the external device against the overlying skin **401.**

As indicated in Fig. 11 to 13, the implant magnet **900** has a north magnetic pole, a south magnetic pole, and as a whole has an overall magnetic dipole moment $m$ that is parallel to the outermost surface **908** and hence parallel to the skin **401** (not shown in Fig. 11 to 13). The overall magnetic dipole moment $m$ need not be precisely parallel to the outermost surface **908** (the skin **401**), but should be at least approximately parallel to it, for example form an angle of 30° or less, preferably 20° or less with respect to said outermost surface **908.**

The implant magnet **900** is rotatable around a rotation axis **907** that in the embodiment shown is perpendicular to said outermost surface **908** so that in each available rotational position of said implant magnet **900** upon rotation around its rotation axis **907,** the overall magnetic dipole moment $m$ is parallel to said outermost surface **908.**

As is seen in Fig. 12 and 13, the implant magnet **900** has an outer end surface **912** facing said outermost surface **908** of said implant device **905** (i.e. towards the skin **401** in the implanted state), and an inner end surface **913** facing away from said outermost surface **908** (i.e. towards the inside of a patient head in the implanted state). Both of the outer and inner end faces **912, 913** are planar surfaces and are perpendicular to said rotation axis **907.** For example, the implant magnet **900** may have a cylindrical disc shape, in which the side face is formed by a cylindrical surface, as shown in Fig. 12. However, the side face need not be precisely cylindrical, but could have a slightly conical shape, as shown in Fig. 13. It is however preferred that said implant magnet **900** has a shape that is rotationally symmetric around said rotation axis **907.**

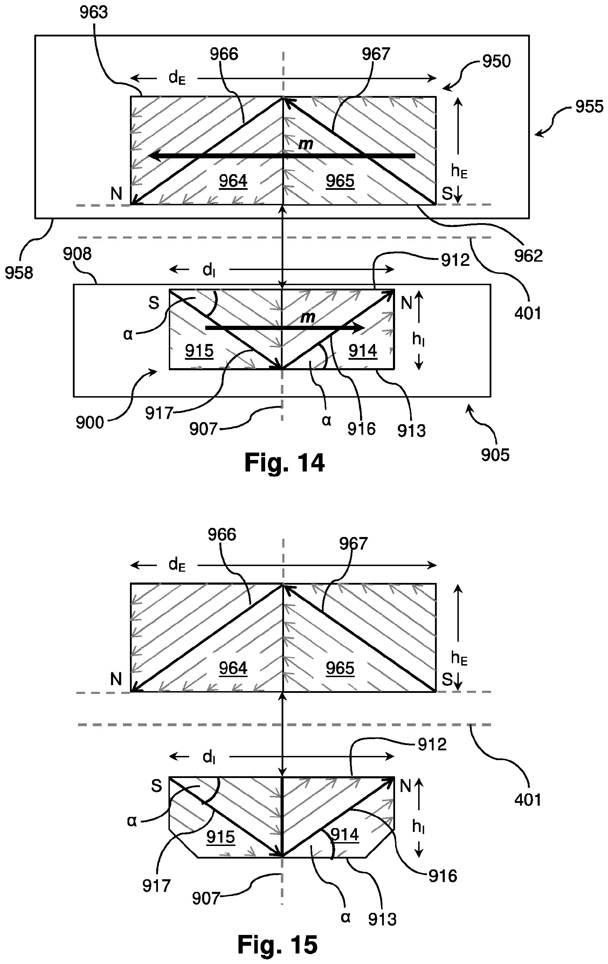

Moreover, the implant magnet **900** has a north end portion **914** including said north magnetic pole and a south end portion **915** including said south magnetic pole. Both of said north and south end portions **914, 915** are formed from permanent magnetic material and each have an individual magnetic dipole moment **916, 917** that is inclined with respect to said overall magnetic dipole moment $m$, as will be explained in more detail with reference to Fig. 14.

Fig. 14 again schematically shows an implant device **905** in a sectional view including an implant magnet **900,** as well as an external device **955** comprising an external magnet **950.** By magnetic interaction between the internal and external magnets **905, 950,** the external device **955** may be attached to the skin **401** of a patient. The internal magnet **900** is comprised of two halves, one being formed by the north end portion **914** and the other by the south end portion **915.** The hatched lines with arrowheads indicate the local magnetization *M.* It is seen that the local magnetizations in the north and south end portions **914, 915** have deviating directions, thereby leading to what was referred to as the "magnetic angle" with reference to the first aspect of the invention above. Each of the north and south end portions **914, 915** has an individual magnetic dipole moment **916, 917,** which corresponds to the space integral over the magnetization *M* in the respective portion. It is then seen that although the overall dipole moment $m$ of the implant magnet **900** as a whole is directed parallel to the outermost surface **908** of the implant device **905,** each of the individual magnetic dipole moments **916, 917** are inclined with respect to the overall dipole moment $m$.

More precisely, it is seen that the magnetic dipole moment **916** in said north end portion **914** is inclined in a plane perpendicular to the outermost surface **908**/skin **401,** to have a component pointing towards said outermost surface **908,** and said individual magnetic dipole moment in said south end portion **915** is inclined to have a component pointing away from said outermost surface. This leads to a situation where the bigger part of the magnetic flux *B* generated by said implant magnet **901** is located to the outside, where it is needed for generating a holding force holding the external magnet **950.**

In particular, in preferred embodiments, when simply regarding the internal magnet **900** in isolation, i.e. without presence of the external magnet **950** and when placed in air or vacuum, at least 55 %, preferably at least 65 % or even 70 % or more of the total magnetic flux **B** of a magnetic field generated outside of the implant magnet **900** is located "outside" of a middle plane arranged at equal distances from the outer and inner end surfaces **912, 913**. Herein, "outside of the middle plane" means the side at which said outermost surface **908** is located in the assembled state. This way, the holding force can be significantly increased over and implant magnet of the same size and material which would be homogeneously magnetized parallel to the skin **401**.

In the embodiment of Fig. 14, the implant magnet **900** has a diameter $d_I$ in a direction parallel to the overall magnetic dipole moment and a thickness $h_I$ in a direction perpendicular to said outermost surface. In view of this geometry, in both of said north and south end portions **914, 915,** said individual magnetic dipole moment **916, 917** is inclined with respect to said overall magnetic dipole moment **m** by an angle $\alpha = arctan (h_I / (d_I /2))$. Herein, the angle $\alpha$ is measured in a plane that is perpendicular to the outermost surface **908**. This choice for the angle $\alpha$ is large enough for providing a significant improvement in attraction force with the external magnet **950** over a prior art implant magnet of same size and material that would be homogeneously magnetized parallel to the skin **401**. A significantly larger angle $\alpha$ has been found to be less favorable, for two reasons. First, for larger angles a, the distance between the north and south poles would decrease, which in turn would lead to an excessive decrease in holding force with increasing distance from the implant magnet **900**. Secondly, the angle $\alpha$ of inclination between each individual magnetic dipole moment **916, 917** in said north and south end portions **914** and **915** with respect to the overall magnetic dipole moment **m** should generally be no more than 60°, and with a certain safety margin, preferably no more than 50°, such as to avoid a situation in which the implant magnet **900** could be inadvertently weakened or demagnetized in a strong external MRI field in case the patient does not hold his or her head straight during the MRI procedure, but for example tilted to one side by e.g. up to 30°. Preferred ranges for the angle $\alpha$ are $arctan (h_I / (d_I /2)) -15° \leq \alpha \leq arctan (h_I / (d_I /2)) + 7°$, more preferably $arctan (h_I / (d_I /2)) -10° \leq \alpha \leq arctan (h_I / (d_I /2)) + 5°$, provided that in each case, preferably $\alpha \leq 50°$.

Note that the external magnet **950** in the external device **955** of the embodiment of Fig. 14 has a similar structure than the internal magnet **905**. That is to say, the external magnet **950** has an overall magnetic dipole moment **m** that is parallel to the innermost surface **958** of the external device **955** that lies adjacent to the skin **401**. The external magnet **950** likewise has a north end portion **964** and a south end portion **965** in which the respective individual magnetic dipole moment **966, 967** is inclined with respect to the overall dipole moment **m,** such that the individual magnetic dipole moment **966** in said north end portion **964** has a component pointing towards the innermost surface **958** of the external device **955,** and such that the individual magnetic dipole moment **967** in said south end portion **965** has a component pointing away from said innermost surface **958** of the external device **955**. However, while the implant magnet **905** has been specifically devised for compatibility with an MRI external magnetic field, the external device **955** can be taken off the cochlear implant user prior to the MRI procedure, such that it does not need to employ a similar design. In particular, the external magnet **950** need not be rotatable, and it is also not necessary that its overall magnetic dipole moment **m** is parallel to the innermost so face **958** (skin **401**). Nevertheless, in preferred embodiments, the design of the external magnet **950** is similar to that of the internal magnet **905,** incorporating some or all of the features that were described in the above summary of the invention.

Fig. 15 shows an embodiment which is similar to that of Fig. 14, with the main difference that the edges of the implant magnet **905** at the inner end surface **913** are chamfered. This allows for an improved magnetic flux, and further allows for concentrating more than half of the magnet mass towards the outside of the middle plane, i.e. on a side of said middle plane at which said outermost surface **908** is located.

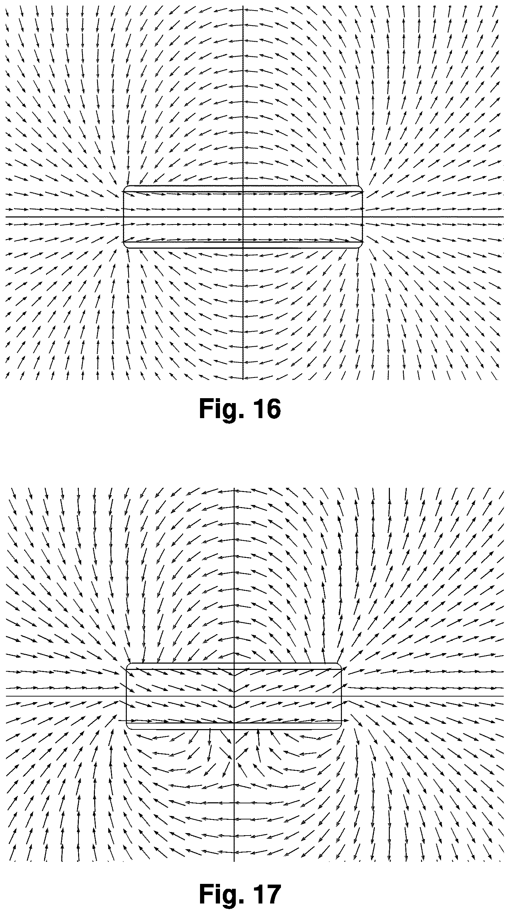

Fig. 16 illustrates the direction of the magnetic flux generated by an ordinary implant magnet that is homogeneously magnetized. In contrast to this, Fig. 17 illustrates the direction of the magnetic flux generated by an implant magnet **900** of the type shown in Fig. 11 to Fig. 14 having north and south end portions **914, 915** with individual magnetic dipole moments **916, 917** that are inclined with respect to the overall magnetic dipole moment of the implant magnet **900**.

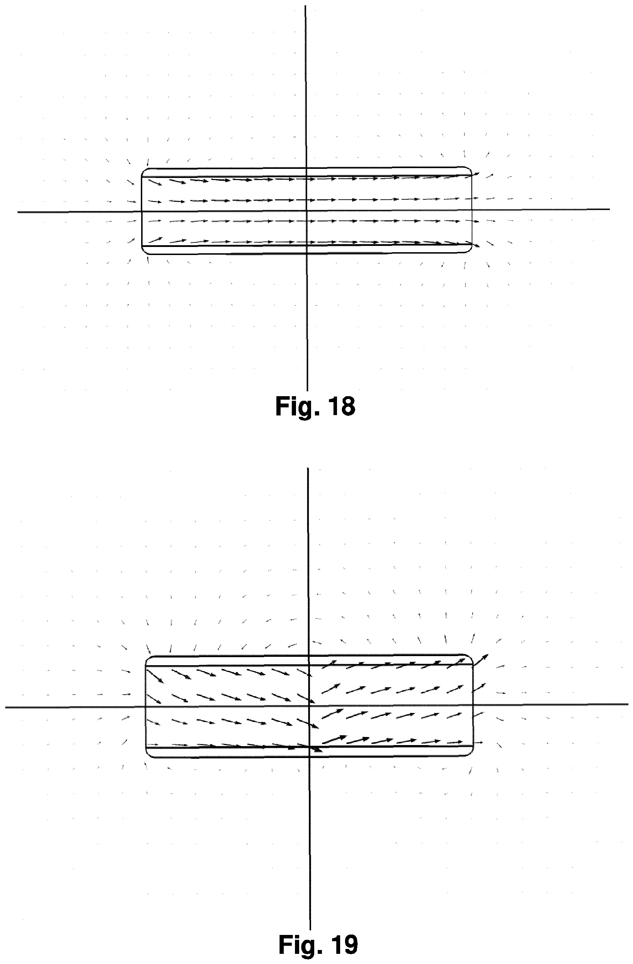

Fig. 18 illustrates the flux density of the ordinary implant magnet of Fig. 16. In other words, while Fig. 16 only shows the direction of the magnetic flux, Fig. 18 indicates the flux density, which is represented by the size of the arrows displayed in the diagram. It is seen that both, the magnetic flux direction and the magnetic flux density are mirror symmetric with respect to the aforementioned middle plane of the magnet, which is a consequence of the homogeneous magnetization. Fig. 19 shows the flux density of the implant magnet **900** of the invention of Fig. 17. It is seen that in the implant magnet **900** of the invention, the poles are shifted "upwards" in the representation of Fig. 19, such that they are located above the middle plane. Indeed, as the skilled person will appreciate, the poles correspond to the regions where the flux density at the surface of the implant magnet **900** is the highest, and these locations are found at the upper left and right corners in the representation of Fig. 19, which is hence consistent with what is schematically shown in Fig. 14 and 15. Moreover, it is seen that of the total magnetic flux of the magnetic field generated outside the implant magnet **900** when taken in isolation, the bigger part is located above the middle plane. Accordingly, the magnetic field generated by the implant magnet **900** of the invention is indeed suitable for generating higher attraction forces when cooperating with an external magnet.

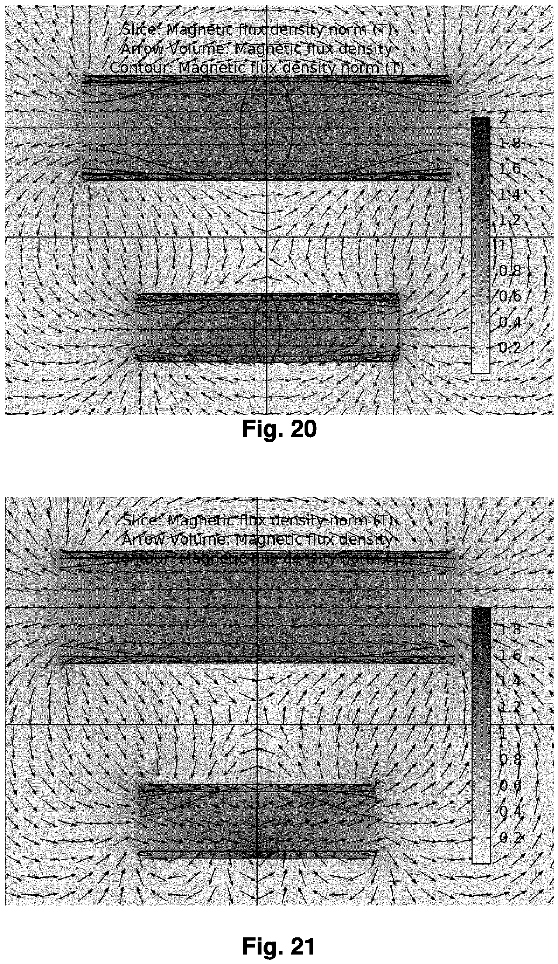

Fig. 20 shows the magnetic flux density generated upon the interaction of a homogeneously magnetized external magnet and the ordinary implant magnet of Fig. 16 and 18. For comparison, Fig. 21 shows the magnetic flux density generated upon the interaction of the same homogeneously magnetized external magnet as in Fig. 20 and the implant magnet **900** of Fig. 17 and 19. It is seen that using the implant magnet **900** of the invention, the attraction force per unit volume of the implant magnet 900 could be increased by 15 %. This gain in attraction force can be even increased further if an external magnet of the type shown under reference sign **950** in Fig. 14 and 15 is used.

In the embodiment shown, both the implant magnet **900** and the external magnet **950** are manufactured from two separate anisotropic magnet pieces which form the north and south end portions **914, 915; 964, 965** in the finished magnet **900, 950**. The anisotropic magnet pieces each have a preferred magnetization direction that corresponds to the direction of the individual magnetic dipole moment **916, 917; 966, 967** in the finished magnet **900, 950**. The preferred magnetization direction can be imprinted on the magnet material by applying a corresponding magnetic field upon its manufacture, for example during a corresponding sintering process. The respective magnet pieces can be joined for example by gluing them together, and only after joining them, the final magnetization is established by applying a strong magnetization pulse parallel to the direction of the overall dipole moment of the finished magnet **900, 950**. Due to the anisotropic character of the magnet pieces, this magnetization pulse will not magnetize both pieces along the direction of the magnetic field of the strong magnetization pulse, but will magnetize them according to their preferred magnetization directions. In preferred embodiments, the implant magnet **900** and/or the external magnet **950** is a rare earth magnet, in particular a rare earth magnet comprising neodymium, samarium, terbium, dysprosium or holmium.

In the embodiments of both, Fig. 14 in Fig. 15, the north and south end portions **914, 915; 964, 965** are directly adjacent with each other, each forming one of two halves of the implant magnet **900** and external magnet **950,** respectively.

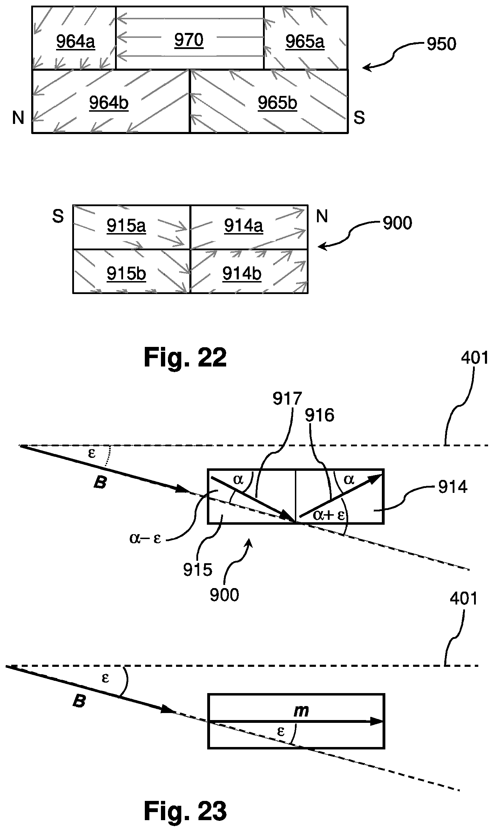

In alternative embodiments, however, north and south end portions of said implant magnet may be separated from each other by an intermediate portion having an individual magnetic dipole moment that is (at least approximately) parallel to said overall magnetic dipole moment. An example for this is shown with respect to the external magnet **950** in Fig. 22, where such intermediate portion is shown at reference sign **970**. This intermediate portion **970** allows for comparatively large inclinations of the magnetization in the north and south end portions **964, 965** while at the same time avoiding magnetic short-circuits at the outer surface, thereby leading to very good holding forces.

In addition or alternatively, one or both of said north and south end portions **914, 915** of said implant magnet **900** may have an outer section **914a, 915a** closer to the outermost surface **908** (not shown in Fig. 22, see Fig. 14) and an inner section **914b, 915b** further away from said outermost surface **908,** and an angle of inclination of the individual magnetic dipole moment (indicated by the magnetization in Fig. 22) with respect to the overall magnetic dipole moment in said outer section **914a, 915a** is less than in said inner section **914b, 915b**. This embodiment allows for an improved magnetic flux within the implant magnet **900** while avoiding the distance of the north and south poles to decrease. A similar design is likewise indicated for the external magnet **950** in Fig. 22.

Fig. 23 shows in the lower half a conventional implant magnet having a homogeneous magnetization and a dipole moment **m** that is parallel to the skin **401** and that is rotatable around an axis perpendicular to the skin **401.** When the implanted person is placed in an external magnetic field **B** of an MRI apparatus, it is generally assumed that the magnetic field **B** is parallel to the skin **401,** and under this assumption, the implant magnet can rotate such as to bring its dipole moment **m** in alignment with the external magnetic field **B,** with no torque acting on the implant magnet anymore. However, this is an idealized assumption, because the implanted magnet might not be perfectly parallel to the skin overlying the magnet, and in practice, the skin overlying the magnet will not be precisely parallel to the external magnetic field **B,** due to the individual anatomy of the patient and the fact that the patient may tilt his or her head sideways. Accordingly, in practice a situation as indicated in the upper part of Fig. 23 arises, in which the magnetic dipole moment **m** of the conventional implant magnet will be inclined with respect to the external magnetic field **B** by an angle $\varepsilon$. In this situation, a torque $\tau$ is applied to the implant magnet having a magnitude $|\tau| = |\boldsymbol{m}| \cdot |\boldsymbol{B}| \cdot \sin(\varepsilon)$.

The upper part of Fig. 23 shows the same situation for an implant magnet **900** according to an embodiment of the invention, having north and south end portions **914, 915** forming halves of the implant magnet **900** and having respective individual magnetic dipole moments **916, 917** which are each inclined with respect to the total magnetic dipole moment of the implant magnet **900** by an angle $\alpha$. This means that in each of the two halves forming the north and south end portions **914, 915,** there will be a local torque applied even if the external magnetic field **B** is parallel to the overall magnetic dipole moment of the implant magnet **900**, but these two local torques cancel each other. Assuming that the magnitude of the individual dipole moment **916, 917** in each of the north and south end portions **914, 916** is $m_o$, the magnitude of the total torque in case of an inclined external magnetic field **B** as illustrated in the lower part of Fig. 23 can be calculated as follows:

$$|\boldsymbol{\tau}| = m_o \cdot |\boldsymbol{B}| \cdot \sin(\alpha + \varepsilon) - m_o \cdot |\boldsymbol{B}| \cdot \sin(\alpha - \varepsilon) = 2 \cdot m_o \cdot |\boldsymbol{B}| \cdot \sin(\varepsilon) \cdot \cos(\alpha).$$

Comparing this torque to the torque experienced by a conventional homogeneously magnetized implant magnet of same dimensions, one can assume that $2 \cdot m_o \approx |\boldsymbol{m}|$, with $|\boldsymbol{m}|$ being again the magnitude of the magnetic dipole moment of the conventional homogeneously magnetized implant magnet. It is therefore seen that the torque experienced by the implant magnet **900** of the invention in the inclined external magnetic field **B** is actually reduced by a factor of cos(a) as compared to the conventional magnet of the same size, making the implant magnet **900** of the invention less sensitive to deviations from the idealized assumption of an external magnetic field being parallel to the skin.

Although various exemplary embodiments of the invention have been disclosed, it should be apparent to those skilled in the art that various changes and modifications can be made which will achieve some of the advantages of the invention without departing from the true scope of the invention.

## Claims

1. An implant device (305) containing signal processing circuitry configured for receiving an implant communications signal transmitted from an external transmitting coil through overlying skin (401) of an implanted patient, wherein the implant device (305) includes an outermost surface (308) adapted to lie between the overlying skin (401) and underlying skull bone of the implanted patient;

   a magnet case (306) within the implant device (305), wherein the magnet case (306) is configured to be rotatable about a case rotation axis (307) which is at least approximately perpendicular to said outermost surface (308) of the implant device (305); and

   an implant magnet arrangement within the magnet case (306) configured to cooperate with an external holding magnet (403) in an external device (402) to be located over the overlying skin (401) to magnetically hold the external device (402) against the overlying skin (401);

   wherein the implant magnet arrangement comprises a plurality of cylindrical magnets (300), each with a center cylinder axis (301) perpendicular to the case rotation axis, and each cylindrical magnet (300) configured to be rotatable about the center cylinder axis;

   wherein each cylindrical magnet of said plurality of cylindrical magnets has an outer cylindrical surface with a north magnetic pole and a south magnetic pole;

   wherein a north magnetic direction (304) is defined by a radial vector extending from the center cylinder axis to the north magnetic pole;

   wherein a south magnetic direction (304) is defined by a radial vector extending from the south magnetic pole to the center cylinder axis (301) ; and

   wherein the north magnetic pole and the south magnetic pole are arranged with respect to each other so as not to lie on a common diameter through the center cylinder axis (301) such that the north magnetic direction (303) and the south magnetic direction (304) form a magnetic angle less than 180 degrees with a vertex at the center cylinder axis (301).

2. The implant device according to claim 1, wherein the implant magnet arrangement is configured for the cylindrical magnets (300) to magnetically align with respect to each other to create a common line of magnetic flux through the cylindrical magnets (300), the magnet case (306), and the overlying skin (401) to cooperate with the external holding magnet (403),
   wherein the magnet arrangement preferably further comprises:

   one or more diametrically magnetized supplemental cylindrical magnets (601) between said plurality of cylindrical magnets (300) configured to couple the common line of magnetic flux between the plurality of cylindrical magnets (300), and/or
   wherein the magnet arrangement preferably further comprises soft magnetic material (701) between the plurality of cylindrical magnets (300) configured to couple the common line of magnetic flux between the plurality of cylindrical magnets (300), and/or wherein said implant device (305) is a hearing implant device, in particular a cochlear implant.

3. The implant device according to one of the preceding claims, wherein the implant magnet arrangement is configured to respond to a strong external magnetic field by rotation of the magnet case (306) about the case rotation axis (307) and rotation of the cylindrical magnets (300) about their respective center cylinder axes (301) so as to minimize net torque imparted to the implant device (305), and/or

   wherein the magnetic angle is between 90 degrees and 140 degrees, and/or
   wherein each cylindrical magnet (300) is configured to be fully rotatable about the center cylinder axis (301) through a complete rotation range of 360 degrees, and/or

wherein each cylindrical magnet (300) is configured to be limitedly rotatable about the center cylinder axis (301) through a limited rotation range of less than 180 degrees, wherein the limited rotation range is preferably 90 degrees.

4. An implant device (905) containing signal processing circuitry configured for receiving an implant communications signal transmitted through overlying skin (401) of an implanted patient, wherein the implant device (905) includes an outermost surface (908) adapted to lie between the overlying skin (401) and underlying skull bone and at least approximately parallel to the skin (401) of the implanted patient; and

an implant magnet (900) configured to cooperate with an external holding magnet (950) in an external device (955) to be located over the overlying skin (401) to magnetically hold the external device (955) against the overlying skin (401);
wherein said implant magnet (900) has a north magnetic pole, a south magnetic pole, and as a whole has an overall magnetic dipole moment that is parallel to or at an angle of 30° or less with respect to said outermost surface (908),
wherein said implant magnet (900) has a north end portion (914) including said north magnetic pole and a south end portion (915) including said south magnetic pole, said north and south end portions (914, 915) each being formed from permanent magnetic material and each having an individual magnetic dipole moment (916, 917) that is inclined with respect to said overall magnetic dipole moment,
wherein said individual magnetic dipole moment (916) in said north end portion (914) is inclined with respect to said overall magnetic dipole moment such as to have a component pointing towards said outermost surface (908), and said individual magnetic dipole moment (917) in said south end portion (915) is inclined with respect to the overall magnetic dipole moment such as to have a component pointing away from said outermost surface (908).

5. The implant device (905) of claim 4, wherein said implant magnet (900) is rotatable around a rotation axis (907) that is perpendicular to said outermost surface (908), or deviates from perpendicular by less than 30°, wherein in each available rotational position of said implant magnet (900) upon rotation around said rotation axis (907), said overall magnetic dipole moment is parallel to or at an angle of 30° or less with respect to said outermost surface (908), and/or wherein said implant device (905) is a hearing implant device, in particular a cochlear implant.

6. The implant device (905) of claim 5, wherein said implant magnet (900) has a shape that is rotationally symmetric around said rotation axis (907), and/or
wherein said implant magnet (900) has an outer end surface (912) facing said outermost surface (908) of said implant device (905) and an inner end surface (913) facing away from said outermost surface (908), wherein one or both of said inner and outer end surfaces (912, 913) are perpendicular to said rotation axis (907).

7. The implant device (905) of one of claims 4 or 5, wherein said implant magnet (900) has a planar outer end surface (912), and/or

wherein the angle of inclination between each individual magnetic dipole moment (916, 917) in said north and south end portions (914, 915) with respect to the overall magnetic dipole moment is $\leq 50°$, and/or
wherein said implant magnet (900) has an average diameter $d_I$ in a direction parallel to the overall magnetic dipole moment and an average thickness $h_I$ in a direction perpendicular to said outermost surface (908), wherein in one or both of said north and south end portions (914, 915), said individual magnetic dipole moment (916, 917) is inclined with respect to said overall magnetic dipole moment by an angle a, wherein

$$arctan\ (h_I\ /\ (d_I\ /2))\ \text{-}15° \leq \alpha \leq arctan\ (h_I\ /\ (d_I\ /2))\ + 7°,$$

preferably

$$arctan\ (h_I\ /\ (d_I\ /2))\ \text{-}10° \leq \alpha \leq arctan\ (h_I\ /\ (d_I\ /2))\ + 5°.$$

8. The implant device (905) of one of claims 4 to 7, wherein said north and south end portions (914, 915) are directly adjacent with each other, and in particular each form one of two halves of said implant magnet (900), and/or

wherein said north and south end portions (914, 915) of said implant magnet (900) are separated from each other by an intermediate portion having an individual magnetic dipole moment that is parallel to said overall magnetic dipole moment, or deviates from parallel by less than 10°, preferably less than 5°, and/or wherein one or both of said north and south end portions (914, 915) of said implant magnet (900) has an outer section (914a, 915a) closer to said outermost surface (908) and an inner section (914b, 915b) further away from said outermost surface (908), wherein an angle of inclination of the individual magnetic dipole moment with respect to the overall magnetic dipole moment in said outer section (914a, 915a) is less than in said inner section (914b, 915b).

9. The implant device (905) of one of claims 4 to 8, wherein said implant magnet (900) has an outer end surface (912) facing said outermost surface (908) of said implant device (905) and an inner end surface (913) facing away from said outermost surface (908), wherein a middle plane is defined to be located at equal distance from said outer and inner end surfaces (912, 913), and wherein said implant magnet (900) fulfils one or both of the following criteria (i) and (ii):

(i) at least 55 %, preferably at least 65 % of the total magnetic flux of a magnetic field generated outside of the implant magnet (900) when placed in isolation in air or a vacuum is located on a side of said middle plane at which said outermost surface (90) is located in the assembled state,

(ii) more than 50%, preferably more than 55% of the mass of the implant magnet (900) is located on a side of said middle plane at which said outermost surface (908) is located, wherein in particular, the edges of the implant magnet (900) at the inner end surface (913) are chamfered, and/or

wherein said north and south end portions (914, 915) are formed from anisotropic magnet elements each having a preferred magnetization direction, wherein said anisotropic magnet elements are joined with each other or with an intermediate portion arranged in between, wherein said preferred magnetization directions are arranged at an angle with respect to the overall dipole moment of the implant magnet (900) as a whole.

10. The implant device (905) of one of claims 4 to 9, wherein on at least part of the inner end surface (913) a layer of magnetic soft material, such as for example soft iron, is applied, and/or wherein said implant magnet (900) is a rare earth magnet, in particular a rare earth magnet comprising neodymium, samarium, terbium, dysprosium or holmium.

11. An implant system comprising an implant device (305, 905) according to one of the preceding claims and an external device (402, 955), said external device (402, 955) comprising signal processing circuitry configured for transmitting an implant communications signal to said implant device (305, 905), said external device (402, 955) comprising an innermost surface (958) adapted to lie adjacent to said skin (401); and an external holding magnet (403, 950) or magnet assembly in said external device (402, 955) to be located over the overlying skin (401) and magnetically configured to cooperate with the implant magnet (900) or with said magnet arrangement of said implant device (305, 905) such as to hold the external device (402, 955) against the skin (401).

12. The implant system of claim 11, wherein said external holding magnet (403, 950) or magnet arrangement has a north magnetic pole, a south magnetic pole, and as a whole has an overall magnetic dipole moment that is parallel to or at an angle of 30° or less with respect to said innermost surface (958) of said external device (402, 955).

13. The implant system of claim 12, wherein said external holding magnet (950) has a north end portion (964) including said north magnetic pole and a south end portion (965) including said south magnetic pole, said north and south end portions (964, 964) each being formed from permanent magnetic material and each having an individual magnetic dipole moment that is inclined with respect to said overall magnetic dipole moment of said external holding magnet (950), wherein said individual magnetic dipole moment (966) in said north end portion (964) has a component pointing towards said innermost surface (958) of said external device (955), and said individual magnetic dipole moment (967) in said south end portion (965) has a component pointing away from said innermost surface (958) of said external device (955).

14. The implant system of one of claims 11 to 13, wherein said external holding magnet (950) is rotatable around a rotation axis that is perpendicular to said innermost surface (958) of said external device (955) or deviates from perpendicular by less than 30°, wherein in each available rotational position of said external holding magnet (950) upon rotation around said rotation axis, said overall magnetic dipole moment is parallel to or at an angle of 30° or less with respect to said innermost surface (958), wherein said external holding magnet (950) preferably has a shape that is rotationally symmetric around its rotation axis, and/or

wherein said north and south end portions (964, 965) of said external holding magnet (950) are directly adjacent with each other, and in particular each form one of two halves of said external holding magnet (950), and/or wherein said external holding magnet (403, 950) is a rare earth magnet, in particular a rare earth magnet comprising neodymium, samarium, terbium, dysprosium or holmium, wherein said external holding magnet (950) has a planar inner end surface (962) facing said innermost surface (958) of said external device (402, 955).

15. The implant system of one of claims 13 or 14, wherein said external holding magnet (950) has an average diameter dE in a direction parallel to the overall magnetic dipole moment and an average thickness hE in a direction perpendicular to said innermost surface (958) of the external device (955), wherein in one or both of said north and south end portions (964, 965), said individual magnetic dipole moment (966, 967) is inclined with respect to said overall magnetic dipole moment by an angle a, wherein

$$arctan\ (h_E\ /\ (d_E\ /2))\ \text{-}15° \leq \alpha \leq arctan\ (h_E\ /\ (d_E\ /2))\ +7°,$$

preferably

$$arctan\ (h_E\ /\ (d_I\ /2))\ \text{-}10° \leq \alpha \leq arctan\ (h_E\ /\ (d_E\ /2))\ +5°,$$

and/or

wherein said north and south end portions (964, 965) of said external holding magnet (950) are separated from each other by an intermediate portion (970) having an individual magnetic dipole moment that is parallel to said overall magnetic dipole moment of said external holding magnet (950) or deviates from parallel by less than 10°, preferably less than 5°, and/or wherein one or both of said north and south end portions (964, 965) of said external holding magnet (950) have an inner section (964a, 965a) closer to said innermost surface (958) of said external device (955) and an outer section (964b, 965b) further away from said innermost surface (958) of said external device (955), wherein an angle of inclination of the individual magnetic dipole moment with respect to the overall magnetic dipole moment in said inner section (964a, 965a) is less than in said outer section (964b, 965b), and/or wherein said external holding magnet (950) has an inner end surface (962) facing said innermost surface (958) of said external device (955) and an outer end surface (963) facing away from said innermost surface (958) of said external device (955), wherein a middle plane is defined to be located at equal distance from said outer and inner end surfaces (963, 962) of said external device (955), and wherein said external holding magnet (950) fulfils one or both of the following criteria (i) and (ii):

(i) at least 55 %, preferably at least 65 % of the total magnetic flux of a magnetic field generated outside of the external holding magnet (950) when placed in isolation in air or a vacuum is located on a side of said middle plane at which said innermost surface of the external device is located in the assembled state, (ii) more than 50%, preferably more than 55% of the mass of the external holding magnet (950) is located on a side of said middle plane at which said innermost surface (958) is located, wherein in particular, the edges of the external holding magnet (950) at the outer end surface are chamfered, and/or

wherein said north and south end portions (964, 965) of said external holding magnet (950) are formed from anisotropic magnet elements each having a preferred magnetization direction, wherein said anisotropic magnet elements are joined with each other or with an intermediate portion arranged in between, wherein said preferred magnetization directions are arranged at an angle with respect to the overall dipole moment of the external holding magnet (950) as a whole.

**Patentansprüche**

1. Implantatvorrichtung (305), die eine Signalverarbeitungsschaltung enthält, die zum Empfangen eines Implantatkommunikationssignals konfiguriert ist, das von einer externen Sendespule durch überlagernde Haut (401) eines implantierten Patienten gesendet wird, wobei die Implantatvorrichtung (305) eine äußerste Oberfläche (308) umfasst, die angepasst ist, um zwischen der überlagernden Haut (401) und dem darunterliegenden Schädelknochen des implantierten Patienten zu liegen;

   ein Magnetgehäuse (306) innerhalb der Implantatvorrichtung (305), wobei das Magnetgehäuse (306) konfiguriert ist, um um eine Gehäusedrehachse (307) drehbar zu sein, die zumindest annähernd senkrecht zu der äußersten Oberfläche (308) der Implantatvorrichtung (305) ist; und
   eine Implantatmagnetanordnung innerhalb des Magnetgehäuses (306), die konfiguriert ist, um mit einem externen Haltemagneten (403) in einer externen Vorrichtung (402) zusammenzuwirken, die über der überlagernden Haut (401) anzuordnen ist, um die externe Vorrichtung (402) magnetisch gegen die überlagernde Haut (401) zu halten;
   wobei die Implantatmagnetanordnung eine Mehrzahl von zylindrischen Magneten (300) umfasst, die jeweils eine zentrale Zylinderachse (301) senkrecht zu der Gehäusedrehachse aufweisen, und jeder zylindrische Magnet (300) konfiguriert ist, um um die zentrale Zylinderachse drehbar zu sein;
   wobei jeder zylindrische Magnet der genannten Mehrzahl von zylindrischen Magneten eine äußere zylindrische Oberfläche mit einem Nordmagnetpol und einem Südmagnetpol aufweist;
   wobei eine Nordmagnetrichtung (304) durch einen radialen Vektor definiert ist, der sich von der zentralen Zylinderachse zu dem Nordmagnetpol erstreckt;
   wobei eine Südmagnetrichtung (304) durch einen radialen Vektor definiert ist, der sich von dem Südmagnetpol zu der zentralen Zylinderachse (301) erstreckt; und
   wobei der Nordmagnetpol und der Südmagnetpol so in Bezug zueinander angeordnet sind, dass sie nicht auf einem gemeinsamen Durchmesser durch die zentrale Zylinderachse (301) liegen, so dass die Nordmagnetrichtung (303) und die Südmagnetrichtung (304) einen Magnetwinkel von weniger als 180 Grad mit einem Scheitelpunkt an der zentralen Zylinderachse (301) bilden.

2. Implantatvorrichtung nach Anspruch 1, wobei die Implantatmagnetanordnung dazu konfiguriert ist, dass sich die zylindrischen Magneten (300) magnetisch in Bezug zueinander ausrichten, um eine gemeinsame Magnetflusslinie durch die zylindrischen Magneten (300), das Magnetgehäuse (306) und die überlagernde Haut (401) zu erzeugen, um mit dem externen Haltemagneten (403) zusammenzuwirken,
   wobei die Magnetanordnung vorzugsweise ferner umfasst:

   einen oder mehrere diametral magnetisierte zusätzliche zylindrische Magneten (601) zwischen der genannten Mehrzahl von zylindrischen Magneten (300), die konfiguriert sind, um die gemeinsame Magnetflusslinie zwischen der Mehrzahl von zylindrischen Magneten (300) zu koppeln, und/oder
   wobei die Magnetanordnung vorzugsweise ferner weichmagnetisches Material (701) zwischen der Mehrzahl von zylindrischen Magneten (300) umfasst, das konfiguriert ist, um die gemeinsame Magnetflusslinie zwischen der Mehrzahl von zylindrischen Magneten (300) zu koppeln, und/oder wobei die genannte Implantatvorrichtung (305) eine Hörimplantatvorrichtung, insbesondere ein Cochleaimplantat, ist.

3. Implantatvorrichtung nach einem der vorhergehenden Ansprüche, wobei die Implantatmagnetanordnung konfiguriert ist, um auf ein starkes externes Magnetfeld durch Drehung des Magnetgehäuses (306) um die Gehäusedrehachse (307) und Drehung der zylindrischen Magneten (300) um ihre jeweiligen zentralen Zylinderachsen (301) zu reagieren, um das der Implantatvorrichtung (305) verliehene Nettodrehmoment zu minimieren, und/oder

   wobei der Magnetwinkel zwischen 90 Grad und 140 Grad liegt, und/oder wobei jeder zylindrische Magnet (300) konfiguriert ist, um vollständig um die zentrale Zylinderachse (301) durch einen vollständigen Drehbereich von 360 Grad drehbar zu sein, und/oder
   wobei jeder zylindrische Magnet (300) konfiguriert ist, um begrenzt um die zentrale Zylinderachse (301) durch einen begrenzten Drehbereich von weniger als 180 Grad drehbar zu sein,
   wobei der begrenzte Drehbereich vorzugsweise 90 Grad beträgt.

4. Implantatvorrichtung (905), die eine Signalverarbeitungsschaltung enthält, die zum Empfangen eines Implantatkommunikationssignals konfiguriert ist, das durch überlagernde Haut (401) eines implantierten Patienten gesendet wird, wobei die Implantatvorrichtung (905) eine äußerste Oberfläche (908) umfasst, die angepasst ist, um zwischen der überlagernden Haut (401) und dem darunterliegenden Schädelknochen und zumindest annähernd parallel zu der Haut (401) des implantierten Patienten zu liegen; und

einen Implantatmagneten (900), der konfiguriert ist, um mit einem externen Haltemagneten (950) in einer externen Vorrichtung (955) zusammenzuwirken, die über der überlagernden Haut (401) anzuordnen ist, um die externe Vorrichtung (955) magnetisch gegen die überlagernde Haut (401) zu halten;
wobei der Implantatmagnet (900) einen Nordmagnetpol und einen Südmagnetpol aufweist und als Ganzes ein magnetisches Gesamtdipolmoment aufweist, das parallel zu oder in einem Winkel von 30° oder weniger in Bezug auf die äußerste Oberfläche (908) ist,
wobei der Implantatmagnet (900) einen Nordendabschnitt (914), der den Nordmagnetpol beinhaltet, und einen Südendabschnitt (915), der den Südmagnetpol beinhaltet, aufweist, wobei der Nord- und der Südendabschnitt (914, 915) jeweils aus permanentmagnetischem Material gebildet sind und jeweils ein individuelles magnetisches Dipolmoment (916, 917) aufweisen, das in Bezug auf das magnetische Gesamtdipolmoment geneigt ist, wobei das individuelle magnetische Dipolmoment (916) in dem Nordendabschnitt (914) in Bezug auf das magnetische Gesamtdipolmoment so geneigt ist, dass es eine Komponente aufweist, die zu der äußersten Oberfläche (908) zeigt, und das individuelle magnetische Dipolmoment (917) in dem Südendabschnitt (915) in Bezug auf das magnetische Gesamtdipolmoment so geneigt ist, dass es eine Komponente aufweist, die von der äußersten Oberfläche (908) weg zeigt.

5. Implantatvorrichtung (905) nach Anspruch 4, wobei der Implantatmagnet (900) um eine Drehachse (907) drehbar ist, die senkrecht zu der äußersten Oberfläche (908) ist oder von der Senkrechten um weniger als 30° abweicht, wobei in jeder verfügbaren Drehposition des Implantatmagneten (900) bei Drehung um die Drehachse (907) das magnetische Gesamtdipolmoment parallel zu oder in einem Winkel von 30° oder weniger in Bezug auf die äußerste Oberfläche (908) ist, und/oder wobei die Implantatvorrichtung (905) eine Hörimplantatvorrichtung, insbesondere ein Cochleaimplantat, ist.

6. Implantatvorrichtung (905) nach Anspruch 5, wobei der Implantatmagnet (900) eine Form aufweist, die rotationssymmetrisch um die Drehachse (907) ist, und/oder wobei der Implantatmagnet (900) eine äußere Endfläche (912), die der äußersten Oberfläche (908) der Implantatvorrichtung (905) zugewandt ist, und eine innere Endfläche (913), die von der äußersten Oberfläche (908) abgewandt ist, aufweist, wobei eine oder beide der inneren und äußeren Endflächen (912, 913) senkrecht zu der Drehachse (907) sind.

7. Implantatvorrichtung (905) nach einem der Ansprüche 4 oder 5, wobei der Implantatmagnet (900) eine planare äußere Endfläche (912) aufweist, und/oder wobei der Neigungswinkel zwischen einem jeden individuellen magnetischen Dipolmoment (916, 917) in dem Nord- und dem Südendabschnitt (914, 915) in Bezug auf das magnetische Gesamtdipolmoment $\leq 50°$ ist, und/oder wobei der Implantatmagnet (900) einen durchschnittlichen Durchmesser $d_I$ in einer Richtung parallel zu dem magnetischen Gesamtdipolmoment und eine durchschnittliche Dicke $h_I$ in einer Richtung senkrecht zu der äußersten Oberfläche (908) aufweist, wobei in einem oder beiden des Nord- und des Südendabschnitts (914, 915) das individuelle magnetische Dipolmoment (916, 917) in Bezug auf das magnetische Gesamtdipolmoment um einen Winkel $\alpha$ geneigt ist, wobei

$$arctan\ (h_I/(d_I/2))\ -15° \leq \alpha \leq arctan\ (h_I/(d_I/2))\ +7°,$$

vorzugsweise

$$arctan\ (h_I/(d_I/2))\ -10° \leq \alpha \leq arctan\ (h_I/(d_I/2))\ +5°.$$

8. Implantatvorrichtung (905) nach einem der Ansprüche 4 bis 7, wobei der Nord- und der Südendabschnitt (914, 915) direkt aneinander angrenzen und insbesondere jeweils eine von zwei Hälften des Implantatmagneten (900) bilden, und/oder

wobei der Nord- und der Südendabschnitt (914, 915) des Implantatmagneten (900) durch einen Zwischenabschnitt voneinander getrennt sind, der ein individuelles magnetisches Dipolmoment aufweist, das parallel zu dem magnetischen Gesamtdipolmoment ist oder von der Parallelen um weniger als 10°, vorzugsweise weniger als 5°, abweicht, und/oder

wobei einer oder beide des Nord- und des Südendabschnitts (914, 915) des Implantatmagneten (900) einen äußeren Abschnitt (914a, 915a) aufweisen, der näher an der äußersten Oberfläche (908) liegt, und einen inneren Abschnitt (914b, 915b), der weiter von der äußersten Oberfläche (908) entfernt ist, wobei ein Neigungswinkel des individuellen magnetischen Dipolmoments in Bezug auf das magnetische Gesamtdipolmoment in dem äußeren Abschnitt (914a, 915a) kleiner ist als in dem inneren Abschnitt (914b, 915b).

9. Implantatvorrichtung (905) nach einem der Ansprüche 4 bis 8, wobei der Implantatmagnet (900) eine äußere Endfläche (912), die der äußersten Oberfläche (908) der Implantatvorrichtung (905) zugewandt ist, und eine innere Endfläche (913), die von der äußersten Oberfläche (908) abgewandt ist, aufweist, wobei eine Mittelebene so definiert ist, dass sie sich in gleichem Abstand von der äußeren und der inneren Endfläche (912, 913) befindet, und wobei der Implantatmagnet (900) eines oder beide der folgenden Kriterien (i) und (ii) erfüllt:

(i) mindestens 55 %, vorzugsweise mindestens 65 % des gesamten Magnetflusses eines Magnetfelds, das außerhalb des Implantatmagneten (900) erzeugt wird, wenn er in Luft oder einem Vakuum isoliert platziert wird, befindet sich auf einer Seite der Mittelebene, an der sich die äußerste Oberfläche (90) im zusammengebauten Zustand befindet,
(ii) mehr als 50 %, vorzugsweise mehr als 55 % der Masse des Implantatmagneten (900) befindet sich auf einer Seite der Mittelebene, an der sich die äußerste Oberfläche (908) befindet, wobei insbesondere die Kanten des Implantatmagneten (900) an der inneren Endfläche (913) abgeschrägt sind, und/oder

wobei der Nord- und der Südendabschnitt (914, 915) aus anisotropen Magnetelementen gebildet sind, die jeweils eine bevorzugte Magnetisierungsrichtung aufweisen, wobei die anisotropen Magnetelemente miteinander oder mit einem dazwischen angeordneten Zwischenabschnitt verbunden sind, wobei die bevorzugten Magnetisierungsrichtungen in einem Winkel in Bezug auf das Gesamtdipolmoment des Implantatmagneten (900) als Ganzes angeordnet sind.

10. Implantatvorrichtung (905) nach einem der Ansprüche 4 bis 9, wobei auf mindestens einem Teil der inneren Endfläche (913) eine Schicht aus weichmagnetischem Material, wie zum Beispiel Weicheisen, aufgebracht ist, und/oder wobei der Implantatmagnet (900) ein Seltenerdmagnet ist, insbesondere ein Seltenerdmagnet, der Neodym, Samarium, Terbium, Dysprosium oder Holmium umfasst.

11. Implantatsystem, umfassend eine Implantatvorrichtung (305, 905) nach einem der vorhergehenden Ansprüche und eine externe Vorrichtung (402, 955), wobei die externe Vorrichtung (402, 955) eine Signalverarbeitungsschaltung umfasst, die zum Senden eines Implantatkommunikationssignals an die Implantatvorrichtung (305, 905) konfiguriert ist, wobei die externe Vorrichtung (402, 955) eine innerste Oberfläche (958) umfasst, die angepasst ist, um angrenzend an die Haut (401) zu liegen; und einen externen Haltemagneten (403, 950) oder eine Magnetanordnung in der externen Vorrichtung (402, 955), die über der überlagernden Haut (401) anzuordnen ist, und magnetisch konfiguriert ist, um mit dem Implantatmagneten (900) oder mit der Magnetanordnung der Implantatvorrichtung (305, 905) zusammenzuwirken, um die externe Vorrichtung (402, 955) gegen die Haut (401) zu halten.

12. Implantatsystem nach Anspruch 11, wobei der externe Haltemagnet (403, 950) oder die Magnetanordnung einen Nordmagnetpol und einen Südmagnetpol aufweist und als Ganzes ein magnetisches Gesamtdipolmoment aufweist, das parallel zu oder in einem Winkel von 30° oder weniger in Bezug auf die innerste Oberfläche (958) der externen Vorrichtung (402, 955) ist.

13. Implantatsystem nach Anspruch 12, wobei der externe Haltemagnet (950) einen Nordendabschnitt (964), der den Nordmagnetpol beinhaltet, und einen Südendabschnitt (965), der den Südmagnetpol beinhaltet, aufweist, wobei der Nord- und der Südendabschnitt (964, 964) jeweils aus permanentmagnetischem Material gebildet sind und jeweils ein individuelles magnetisches Dipolmoment aufweisen, das in Bezug auf das magnetische Gesamtdipolmoment des externen Haltemagneten (950) geneigt ist, wobei das individuelle magnetische Dipolmoment (966) in dem Nordendabschnitt (964) eine Komponente aufweist, die zu der innersten Oberfläche (958) der externen Vorrichtung (955) zeigt, und das individuelle magnetische Dipolmoment (967) in dem Südendabschnitt (965) eine Komponente aufweist, die von der innersten Oberfläche (958) der externen Vorrichtung (955) weg zeigt.

14. Implantatsystem nach einem der Ansprüche 11 bis 13, wobei der externe Haltemagnet (950) um eine Drehachse drehbar ist, die senkrecht zu der innersten Oberfläche (958) der externen Vorrichtung (955) ist oder von der Senkrechten um weniger als 30° abweicht, wobei in jeder verfügbaren Drehposition des externen Haltemagneten (950) bei Drehung um die Drehachse das magnetische Gesamtdipolmoment parallel zu oder in einem Winkel von 30° oder weniger in Bezug auf die innerste Oberfläche (958) ist, wobei der externe Haltemagnet (950) vorzugsweise eine Form aufweist, die rotationssymmetrisch um seine Drehachse ist, und/oder

wobei der Nord- und der Südendabschnitt (964, 965) des externen Haltemagneten (950) direkt aneinander angrenzen und insbesondere jeweils eine von zwei Hälften des externen Haltemagneten (950) bilden, und/oder wobei der externe Haltemagnet (403, 950) ein Seltenerdmagnet ist, insbesondere ein Seltenerdmagnet, der Neodym, Samarium, Terbium, Dysprosium oder Holmium umfasst, wobei der externe Haltemagnet (950) eine planare innere Endfläche (962) aufweist, die der innersten Oberfläche (958) der externen Vorrichtung (402, 955) zugewandt ist.

15. Implantatsystem nach einem der Ansprüche 13 oder 14, wobei der externe Haltemagnet (950) einen durchschnittlichen Durchmesser $d_E$ in einer Richtung parallel zu dem magnetischen Gesamtdipolmoment und eine durchschnittliche Dicke $h_E$ in einer Richtung senkrecht zu der innersten Oberfläche (958) der externen Vorrichtung (955) aufweist, wobei in einem oder beiden des Nord- und des Südendabschnitts (964, 965) das individuelle magnetische Dipolmoment (966, 967) in Bezug auf das magnetische Gesamtdipolmoment um einen Winkel $\alpha$ geneigt ist, wobei

$$arctan\ (h_E\,/(d_E\,/2))\ \text{-}15° \le \alpha \le arctan\ (h_E\,/(d_E\,/2))\ + 7°,$$

*vorzugsweise*

$$arctan\ (h_E\,/(d_E\,/2))\ \text{-}10° \le \alpha \le arctan\ (h_E\,/(d_E\,/2))\ + 5°,$$

*und/oder*

wobei der Nord- und der Südendabschnitt (964, 965) des externen Haltemagneten (950) durch einen Zwischenabschnitt (970) voneinander getrennt sind, der ein individuelles magnetisches Dipolmoment aufweist, das parallel zu dem magnetischen Gesamtdipolmoment des externen Haltemagneten (950) ist oder von der Parallelen um weniger als 10°, vorzugsweise weniger als 5°, abweicht, und/oder wobei einer oder beide des Nord- und des Südendabschnitts (964, 965) des externen Haltemagneten (950) einen inneren Abschnitt (964a, 965a) aufweisen, der näher an der innersten Oberfläche (958) der externen Vorrichtung (955) liegt, und einen äußeren Abschnitt (964b, 965b), der weiter von der innersten Oberfläche (958) der externen Vorrichtung (955) entfernt ist, wobei ein Neigungswinkel des individuellen magnetischen Dipolmoments in Bezug auf das magnetische Gesamtdipolmoment in dem inneren Abschnitt (964a, 965a) kleiner ist als in dem äußeren Abschnitt (964b, 965b), und/oder wobei der externe Haltemagnet (950) eine innere Endfläche (962), die der innersten Oberfläche (958) der externen Vorrichtung (955) zugewandt ist, und eine äußere Endfläche (963), die von der innersten Oberfläche (958) der externen Vorrichtung (955) abgewandt ist, aufweist, wobei eine Mittelebene so definiert ist, dass sie sich in gleichem Abstand von der äußeren und der inneren Endfläche (963, 962) der externen Vorrichtung (955) befindet, und wobei der externe Haltemagnet (950) eines oder beide der folgenden Kriterien (i) und (ii) erfüllt:

(i) mindestens 55 %, vorzugsweise mindestens 65 % des gesamten Magnetflusses eines Magnetfelds, das außerhalb des externen Haltemagneten (950) erzeugt wird, wenn er in Luft oder einem Vakuum isoliert platziert wird, befindet sich auf einer Seite der Mittelebene, an der sich die innerste Oberfläche der externen Vorrichtung im zusammengebauten Zustand befindet,

(ii) mehr als 50 %, vorzugsweise mehr als 55 % der Masse des externen Haltemagneten (950) befindet sich auf einer Seite der Mittelebene, auf der sich die innerste Oberfläche (958) befindet, wobei insbesondere die Kanten des externen Haltemagneten (950) an der äußeren Endfläche abgeschrägt sind, und/oder

wobei der Nord- und der Südendabschnitt (964, 965) des externen Haltemagneten (950) aus anisotropen Magnetelementen gebildet sind, die jeweils eine bevorzugte Magnetisierungsrichtung aufweisen, wobei die anisotropen Magnetelemente miteinander oder mit einem dazwischen angeordneten Zwischenabschnitt verbunden sind, wobei die bevorzugten Magnetisierungsrichtungen in einem Winkel in Bezug auf das Gesamtdipolmoment des externen Haltemagneten (950) als Ganzes angeordnet sind.

**Revendications**

1. Dispositif d'implant (305) contenant un ensemble de circuits de traitement de signaux configuré pour recevoir un signal de communication d'implant transmis à partir d'une bobine de transmission externe à travers la peau sus-jacente (401) d'un patient implanté, dans lequel le dispositif d'implant (305) inclut une surface la plus extérieure (308) adaptée pour se trouver entre la peau sus-jacente (401) et l'os crânien sous-jacent du patient implanté ;

   un boîtier pour aimants (306) dans le dispositif d'implant (305), dans lequel le boîtier pour aimants (306) est configuré pour pouvoir tourner autour d'un axe de rotation de boîtier (307) qui est au moins approximativement perpendiculaire à ladite surface la plus extérieure (308) du dispositif d'implant (305) ; et
   un agencement d'aimants d'implant dans le boîtier pour aimants (306) configuré pour coopérer avec un aimant de maintien externe (403) dans un dispositif externe (402) devant être positionné sur la peau sus-jacente (401) pour maintenir magnétiquement le dispositif externe (402) contre la peau sus-jacente (401) ;
   dans lequel l'agencement d'aimants d'implant comprend une pluralité d'aimants cylindriques (300), chacun avec un axe de cylindre central (301) perpendiculaire à l'axe de rotation de boîtier, et chaque aimant cylindrique (300) étant configuré pour pouvoir tourner autour de l'axe de cylindre central ;
   dans lequel chaque aimant cylindrique de ladite pluralité d'aimants cylindriques a une surface cylindrique externe avec un pôle magnétique nord et un pôle magnétique sud ; dans lequel une direction magnétique nord (304) est définie par un vecteur radial s'étendant de l'axe de cylindre central au pôle magnétique nord ;
   dans lequel une direction magnétique sud (304) est définie par un vecteur radial s'étendant du pôle magnétique sud à l'axe de cylindre central (301) ; et
   dans lequel le pôle magnétique nord et le pôle magnétique sud sont agencés l'un par rapport à l'autre de sorte à ne pas se trouver sur un diamètre commun à travers l'axe de cylindre central (301) de telle sorte que la direction magnétique nord (303) et la direction magnétique sud (304) forment un angle magnétique inférieur à 180 degrés avec un sommet au niveau de l'axe de cylindre central (301).

2. Dispositif d'implant selon la revendication 1, dans lequel l''agencement d'aimants d'implant est configuré pour que les aimants cylindriques (300) s'alignent magnétiquement les uns par rapport aux autres pour créer une ligne commune de flux magnétique à travers les aimants cylindriques (300), le boîtier pour aimants (306) et la peau sus-jacente (401) pour coopérer avec l'aimant de maintien externe (403),
   dans lequel l'agencement d'aimants comprend en outre de préférence :

   un ou plusieurs aimants cylindriques complémentaires diamétralement aimantés (601) entre ladite pluralité d'aimants cylindriques (300) configurés pour coupler la ligne commune de flux magnétique entre la pluralité d'aimants cylindriques (300), et/ou
   dans lequel l'agencement d'aimants comprend en outre de préférence un matériau magnétique doux (701) entre la pluralité d'aimants cylindriques (300) configuré pour coupler la ligne commune de flux magnétique entre la pluralité d'aimants cylindriques (300), et/ou dans lequel ledit dispositif d'implant (305) est un dispositif d'implant auditif, en particulier un implant cochléaire.

3. Dispositif d'implant selon l'une des revendications précédentes, dans lequel l'agencement d'aimants d'implant est configuré pour répondre à un champ magnétique externe fort par rotation du boîtier d'aimants (306) autour de l'axe de rotation de boîtier (307) et par rotation des aimants cylindriques (300) autour de leurs axes de cylindre centraux (301) respectifs de sorte à minimiser le couple net conféré au dispositif d'implant (305), et/ou

   dans lequel l'angle magnétique est compris entre 90 et 140 degrés, et/ou
   dans lequel chaque aimant cylindrique (300) est configuré pour pouvoir tourner complètement autour de l'axe de cylindre central (301) sur une plage de rotation complète de 360 degrés, et/ou
   dans lequel chaque aimant cylindrique (300) est configuré pour pouvoir tourner de manière limitée autour de l'axe de cylindre central (301) sur une plage de rotation limitée inférieure à 180 degrés, dans lequel la plage de rotation limitée est de préférence de 90 degrés.

4. Dispositif d'implant (905) contenant un ensemble de circuits de traitement de signaux configuré pour recevoir un signal de communication d'implant transmis à travers la peau sus-jacente (401) d'un patient implanté, dans lequel le dispositif d'implant (905) inclut une surface la plus extérieure (908) adaptée pour se trouver entre la peau sus-jacente (401) et l'os crânien sousjacent et au moins approximativement parallèle à la peau (401) du patient implanté ; et

un aimant d'implant (900) configuré pour coopérer avec un aimant de maintien externe (950) dans un dispositif externe (955) devant être situé sur la peau sus-jacente (401) pour maintenir magnétiquement le dispositif externe (955) contre la peau sus-jacente (401) ;

dans lequel ledit aimant d'implant (900) a un pôle magnétique nord, un pôle magnétique sud, et dans son ensemble, a un moment magnétique dipolaire global qui est parallèle à ladite surface la plus extérieure (908) ou qui forme un angle inférieur ou égal à 30° par rapport à celle-ci,

dans lequel ledit aimant d'implant (900) a une partie d'extrémité nord (914) incluant ledit pôle magnétique nord et une partie d'extrémité sud (915) incluant ledit pôle magnétique sud, lesdites parties d'extrémité nord et sud (914, 915) étant chacune formée à partir d'un matériau magnétique permanent et chacune ayant un moment magnétique dipolaire individuel (916, 917) qui est incliné par rapport audit moment magnétique dipolaire global, dans lequel ledit moment magnétique dipolaire individuel (916) dans ladite partie d'extrémité nord (914) est incliné par rapport audit moment magnétique dipolaire global de sorte à avoir une composante pointant vers ladite surface la plus extérieure (908), et ledit moment magnétique dipolaire individuel (917) dans ladite partie d'extrémité sud (915) est incliné par rapport au moment magnétique dipolaire global de sorte à avoir une composante pointant à l'opposé de ladite surface la plus extérieure (908).

5. Dispositif d'implant (905) selon la revendication 4, dans lequel ledit aimant d'implant (900) est rotatif autour d'un axe de rotation (907) qui est perpendiculaire à ladite surface la plus extérieure (908), ou dévie de la perpendiculaire de moins de 30°, dans lequel dans chaque position de rotation disponible dudit aimant d'implant (900) lors de la rotation autour dudit axe de rotation (907), ledit moment magnétique dipolaire global est parallèle à ladite surface la plus extérieure (908) ou forme un angle inférieur ou égal à 30° par rapport à celle-ci, et/ou dans lequel ledit dispositif d'implant (905) est un dispositif d'implant auditif, en particulier un implant cochléaire.

6. Dispositif d'implant (905) selon la revendication 5, dans lequel ledit aimant d'implant (900) a une forme qui est symétrique en rotation autour dudit axe de rotation (907), et/ou
   dans lequel ledit aimant d'implant (900) a une surface d'extrémité externe (912) faisant face à ladite surface la plus extérieure (908) dudit dispositif d'implant (905) et une surface d'extrémité interne (913) opposée à ladite surface la plus extérieure (908), dans lequel une ou les deux desdites surfaces d'extrémité interne et externe (912, 913) sont perpendiculaires audit axe de rotation (907).

7. Dispositif d'implant (905) selon l'une des revendications 4 ou 5, dans lequel ledit aimant d'implant (900) a une surface d'extrémité externe plane (912), et/ou dans lequel l'angle d'inclinaison entre chaque moment magnétique dipolaire individuel (916, 917) dans lesdites parties d'extrémité nord et sud (914, 915) par rapport au moment magnétique dipolaire global est ≤ 50°, et/ou
   dans lequel ledit aimant d'implant (900) a un diamètre moyen $d_I$ dans une direction parallèle au moment magnétique dipolaire global et une épaisseur moyenne $h_I$ dans une direction perpendiculaire à ladite surface la plus extérieure (908), dans lequel dans l'une ou les deux desdites parties d'extrémité nord et sud (914, 915), ledit moment magnétique dipolaire individuel (916, 917) est incliné par rapport audit moment magnétique dipolaire global d'un angle α, dans lequel

$$arctan\ (h_I/(d_I/2)) - 15° \le \alpha \le arctan\ (h_I/(d_I/2)) + 7°,$$

de préférence

$$arctan\ (h_I/(d_I/2)) - 10° \le \alpha \le arctan\ (h_I/(d_I/2)) + 5°.$$

8. Dispositif d'implant (905) selon l'une des revendications 4 à 7, dans lequel lesdites parties d'extrémité nord et sud (914, 915) sont directement adjacentes l'une à l'autre, et en particulier, chacune forme une ou deux moitiés dudit aimant d'implant (900), et/ou dans lequel lesdites parties d'extrémité nord et sud (914, 915) dudit aimant d'implant (900) sont séparées l'une de l'autre par une partie intermédiaire ayant un moment magnétique dipolaire individuel qui est parallèle audit moment magnétique dipolaire global, ou dévie de la parallèle de moins de 10°, de préférence de moins de 5°, et/ou dans lequel l'une ou les deux desdites parties d'extrémité nord et sud (914, 915) dudit aimant d'implant (00) a une région externe (914a, 915a) plus proche de ladite surface la plus extérieure (908) et une section interne (914b, 915b) plus éloignée de ladite surface la plus extérieure (908), dans lequel un angle d'inclinaison du moment magnétique dipolaire individuel par rapport au moment magnétique dipolaire global dans ladite section externe (914a, 915a) est inférieur dans ladite section interne (914b, 915b).

**9.** Dispositif d'implant (905) selon l'une des revendications 4 à 8, dans lequel ledit aimant d'implant (900) a une surface d'extrémité externe (912) faisant face à ladite surface la plus extérieure (908) dudit dispositif d'implant (905) et une surface d'extrémité interne (913) opposée à ladite surface la plus extérieure (908), dans lequel un plan médian est défini comme étant situé à égale distance desdites surfaces d'extrémité externe et interne (912, 913), et dans lequel ledit aimant d'implant (900) remplit l'un des critères (i) et (ii) suivants ou les deux :

(i) au moins 55 %, de préférence au moins 65 % du flux magnétique total d'un champ magnétique généré à l'extérieur de l'aimant d'implant (900) lorsqu'il est placé de manière isolée dans de l'air ou un vide est situé sur un côté dudit plan médian au niveau duquel ladite surface la plus extérieure (90) est située dans l'état assemblé,

(ii) plus de 50 %, de préférence plus de 55 % de la masse de l'aimant d'implant (900) est située sur un côté dudit plan médian au niveau duquel ladite surface la plus extérieure (908) est située, dans lequel en particulier, les bords de l'aimant d'implant (900) au niveau de la surface d'extrémité interne (913) sont chanfreinés, et/ou

dans lequel lesdites parties d'extrémité nord et sud (914, 915) sont formées à partir d'éléments d'aimant anisotropes ayant chacun une direction d'aimantation préférée, dans lequel lesdits éléments d'aimant anisotropes sont joints les uns aux autres ou avec une partie intermédiaire agencée entre eux, dans lequel lesdites directions d'aimantation préférées sont agencées selon un angle par rapport au moment dipolaire global de l'aimant d'implant (900) dans son ensemble.

**10.** Dispositif d'implant (905) selon l'une des revendications 4 à 9, dans lequel sur au moins une partie de la surface d'extrémité interne (913) une couche de matériau magnétique doux, tel que par exemple du fer doux, est appliquée, et/ou

dans lequel ledit aimant d'implant (900) est un aimant de terres rares, en particulier un aimant de terres rares comprenant du néodyme, du samarium, du terbium, du dysprosium ou de l'holmium.

**11.** Système d'implant comprenant un dispositif d'implant (305, 905) selon l'une des revendications précédentes et un dispositif externe (402, 955), ledit dispositif externe (402, 955) comprenant un ensemble de circuits de traitement de signaux configuré pour transmettre un signal de communication d'implant audit dispositif d'implant (305, 905), ledit dispositif externe (402, 955) comprenant une surface la plus extérieure (958) adaptée pour se trouver adjacente à ladite peau (401) ; et un aimant de maintien externe (403, 950) ou ensemble d'aimants dans ledit dispositif externe (402, 955) devant être situé sur la peau sus-jacente (401) et configuré magnétiquement pour coopérer avec l'aimant d'implant (900) ou avec ledit agencement d'aimants dudit dispositif d'implant (305, 905) de sorte à maintenir le dispositif externe (402, 955) contre la peau (401).

**12.** Système d'implant selon la revendication 11, dans lequel ledit aimant de maintien externe (403, 950) ou agencement d'aimants a un pôle magnétique nord, un pôle magnétique sud et dans son ensemble, a un moment magnétique dipolaire global qui est parallèle à ladite surface la plus intérieure (958) dudit dispositif externe (402, 955) ou selon un angle inférieur ou égal à 30° par rapport à celle-ci.

**13.** Système d'implant selon la revendication 12, dans lequel ledit aimant de maintien externe (950) a une partie d'extrémité nord (964) incluant ledit pôle magnétique nord et une partie d'extrémité sud (965) incluant ledit pôle magnétique sud, lesdites parties d'extrémité nord et sud (964, 964) étant chacune formée à partir de matériau magnétique permanent et chacune ayant un moment magnétique dipolaire individuel qui est incliné par rapport audit moment magnétique dipolaire global dudit aimant de maintien externe (950),

dans lequel ledit moment magnétique dipolaire individuel (966) dans ladite partie d'extrémité nord (964) a une composante pointant vers ladite surface la plus intérieure (958) dudit dispositif externe (955), et ledit moment magnétique dipolaire individuel (967) dans ladite partie d'extrémité sud (965) a une composante pointant à l'opposé de ladite surface la plus intérieure (958) dudit dispositif externe (955).

**14.** Système d'implant selon l'une des revendications 11 à 13, dans lequel ledit aimant de maintien externe (950) est rotatif autour d'un axe de rotation qui est perpendiculaire à ladite surface la plus intérieure (958) dudit dispositif externe (955) ou dévie de la perpendiculaire de moins de 30°, dans lequel dans chaque position de rotation disponible dudit aimant de maintien externe (950) lors de la rotation autour dudit axe de rotation, ledit moment magnétique dipolaire global est parallèle à ladite surface la plus intérieure (958) ou selon un angle inférieur ou égal à 30° par rapport à celle-ci, dans lequel ledit aimant de maintien externe (950) a de préférence une forme qui est symétrique en rotation autour de son axe de rotation, et/ou

dans lequel lesdites parties d'extrémité nord et sud (964, 965) dudit aimant de maintien externe (950) sont directement adjacentes l'une à l'autre, et en particulier, chacune forme l'une de deux moitiés dudit aimant de maintien externe (950), et/ou

dans lequel ledit aimant de maintien externe (403, 950) est un aimant de terres rares, en particulier un aimant de terres rares comprenant du néodyme, du samarium, du terbium, du dysprosium ou de l'holmium,

dans lequel ledit aimant de maintien externe (950) a une surface d'extrémité interne plane (962) faisant face à ladite surface la plus intérieure (958) dudit dispositif externe (402, 955).

15. Système d'implant selon l'une des revendications 13 ou 14, dans lequel ledit aimant de maintien externe (950) a un diamètre moyen $d_E$ dans une direction parallèle au moment magnétique dipolaire global et une épaisseur moyenne $h_E$ dans une direction perpendiculaire à ladite surface la plus intérieure (958) du dispositif externe (955), dans lequel dans l'une ou les deux desdites parties d'extrémité nord et sud (964, 965), ledit moment magnétique dipolaire individuel (966, 967) est incliné par rapport audit moment magnétique dipolaire global d'un angle $\alpha$, dans lequel

$$arctan\ (h_E/(d_E/2)) - 15° \leq \alpha \leq arctan\ (h_E/(d_E/2)) + 7°,$$

de préférence

$$arctan\ (h_E/\ (d_I/2)) - 10° \leq \alpha \leq arctan\ (h_E/(d_E/2)) + 5°,$$

et/ou

dans lequel lesdites parties d'extrémité nord et sud (964, 965) dudit aimant de maintien externe (950) sont séparées l'une de l'autre par une partie intermédiaire (970) ayant un moment magnétique dipolaire individuel qui est parallèle audit moment magnétique dipolaire global dudit aimant de maintien externe (950) ou s'écarte de la parallèle de moins de 10°, de préférence de moins de 5°, et/ou

dans lequel l'une ou les deux desdites parties d'extrémité nord et sud (964, 965) dudit aimant de maintien externe (950) ont une section interne (964a, 965a) plus près de ladite surface la plus intérieure (958) dudit dispositif externe (955) et une section externe (964b, 965b) plus éloignée de ladite surface la plus intérieure (958) dudit dispositif externe (955), dans lequel un angle d'inclinaison du moment magnétique dipolaire individuel par rapport au moment magnétique dipolaire global dans ladite section interne (964a, 965a) est inférieur dans ladite section externe (964b, 965b), et/ou

dans lequel ledit aimant de maintien externe (950) a une surface d'extrémité interne (962) faisant face à ladite surface la plus intérieure (958) dudit dispositif externe (955) et une surface d'extrémité externe (963) opposée à ladite surface la plus intérieure (958) dudit dispositif externe (955),

dans lequel un plan médian est défini comme étant situé à égale distance desdites surfaces d'extrémité externe et interne (963,962) dudit dispositif externe (955), et dans lequel ledit aimant de maintien externe (950) remplit l'un des critères (i) et (ii) suivants ou les deux :

(i) au moins 55 %, de préférence au moins 65 % du flux magnétique total d'un champ magnétique généré à l'extérieur de l'aimant de maintien externe (950) lorsqu'il est placé de manière isolée dans de l'air ou un vide est situé sur un côté dudit plan médian au niveau duquel ladite surface la plus intérieure du dispositif externe est située dans l'état assemblé,

(ii) plus de 50 %, de préférence plus de 55 % de la masse de l'aimant de maintien externe (950) est située sur un côté dudit plan médian au niveau duquel ladite surface la plus intérieure (958) est située, dans lequel en particulier, les bords de l'aimant de maintien externe (950) au niveau de la surface d'extrémité externe sont chanfreinés, et/ou

dans lequel lesdites parties d'extrémité nord et sud (964, 965) dudit aimant de maintien externe (950) sont formées à partir d'éléments d'aimant anisotropes ayant chacun une direction d'aimantation préférée, dans lequel lesdits éléments d'aimant anisotropes sont joints les uns aux autres ou avec une partie intermédiaire agencée entre eux, dans lequel lesdites directions d'aimantation préférées sont agencées selon un angle par rapport au moment dipolaire global de l'aimant de maintien externe (950) dans son ensemble.

**Fig. 1**

**Fig. 2**

300 311

301 302

S α N

304 303

309 310

**Fig. 3A**

307

308 306

300 301

301 305

**Fig. 3B**

**Fig. 4A**

**Fig. 4B**

501

300    300

304    304

303    303

501

**Fig. 5A**

501

401

305

300

501

**Fig. 5B**

**Fig. 6A**

**Fig. 6B**

**Fig. 7A**

**Fig. 7B**

**Fig. 8**

**Fig. 9A**

**Fig. 9B**

**Fig. 9C**

EP 3 964 259 B1

Fig. 10A

Fig. 10B

Fig. 10C

33

**Fig. 11**

**Fig. 12**

**Fig. 13**

**Fig. 14**

**Fig. 15**

Fig. 16

Fig. 17

**Fig. 18**

**Fig. 19**

**Fig. 20**

**Fig. 21**

964a  970  965a  950

964b  965b

N  S

S  915a  914a  N  900

915b  914b

## Fig. 22

401

917
916

$\varepsilon$

*B*

$\alpha$  $\alpha$  914

$\alpha + \varepsilon$

$\alpha - \varepsilon$

915

900  401

$\varepsilon$

*B*

*m*

$\varepsilon$

## Fig. 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7566296 B
- US 20110022120 A
- US 8634909 B
- WO 2017105510 A
- US 9872993 B2
- KR 20200078292 A
- US 10532209 B2
- EP 2560730 A1